# EUROPEAN PATENT SPECIFICATION

(11) **EP 1 549 677 B1**
(45) Date of publication and mention of the grant of the patent: **13.04.2011**
(21) Application number: 03747838.5
(22) Date of filing: 26.09.2003
(51) Int. Cl.: C07K 14/745

(54) **FVII OR FVIIa VARIANTS HAVING INCREASED CLOTTING ACTIVITY**
FVII- ODER FVIIa-VARIANTEN MIT ERHÖHTER KOAGULATIONSWIRKUNG
VARIANTS FVII OU FVIIa PRESENTANT UNE ACTIVITE DE COAGULATION AMELIOREE

(30) Priority: 30.09.2002 US 414836 P; 19.06.2003 US 479642 P
(43) Date of publication of application: 06.07.2005
(73) Proprietor: Bayer HealthCare LLC, Berkeley CA 94710 (US)
(72) Inventor: HAANING, Jesper, Mortensen, DK-3460 Birkeroed (DK); ANDERSEN, Kim, Vilbour, DK-2700 Broenshoej (DK)
(74) Representative: Carpmaels & Ransford
(86) International application number: PCT/DK2003/000632
(87) International publication number: WO 2004/029091

(56) References cited:
- WO-A-00/66753
- WO-A-01/58935
- WO-A-02/38162
- WO-A-94/27631
- WO-A-03/093465
- CHEUNG WING-FAI ET AL: "Localization of a metal-dependent epitope to the amino terminal residues 33-40 of human factor IX" THROMBOSIS RESEARCH, vol. 80, no. 5, 1995, pages 419-427, XP002273438 ISSN: 0049-3848
- DICKINSON CRAIG D ET AL: "Identification of surface residues mediating tissue factor binding and catalytic function of the serine protease factor VIIa" PROCEEDINGS OF THE NATIONAL ACADEMY OF SCIENCES OF THE UNITED STATES, vol. 93, no. 25, 1996, pages 14379-14384, XP002273439 1996 ISSN: 0027-8424
- DICKINSON CRAIG D ET AL: "Active site modification of factor VIIa affects interactions of the protease domain with tissue factor" JOURNAL OF BIOLOGICAL CHEMISTRY, vol. 272, no. 32, 1997, pages 19875-19879, XP002273440 ISSN: 0021-9258
- SORENSEN BRIT BINOW ET AL: "Incorporation of an active site inhibitor in factor VIIa alters the affinity for tissue factor" JOURNAL OF BIOLOGICAL CHEMISTRY, vol. 272, no. 18, 1997, pages 11863-11868, XP002273441 ISSN: 0021-9258
- PETROVAN RAMONA J ET AL: "Residue Met156 contributes to the labile enzyme conformation of coagulation factor VIIa" JOURNAL OF BIOLOGICAL CHEMISTRY, vol. 276, no. 9, 2 March 2001 (2001-03-02), pages 6616-6620, XP002273442 ISSN: 0021-9258
- SAMPATH SRIDHARA ET AL: "Activation of a recombinant human factor VII structural analogue alters its affinity of binding to tissue factor" AMERICAN JOURNAL OF HEMOTOLOGY, vol. 53, 1996, pages 66-71, XP002273443
- HIGASHI SHOUICHI ET AL: "Molecular mechanism of tissue factor-mediated acceleration of factor VIIa activity" JOURNAL OF BIOLOGICAL CHEMISTRY, vol. 271, no. 43, 1996, pages 26569-26574, XP002273444 ISSN: 0021-9258

## Description

### FIELD OF THE INVENTION

The present invention relates to novel FVII or FVIIa variants comprising the substitution L65Q. Such variants exhibit increased clotting activity. The present invention also relates to use of such polypeptide variants in therapy, in particular for the treatment of a variety of coagulation-related disorders.

### BACKGROUND OF THE INVENTION

Blood coagulation is a process consisting of a complex interaction of various blood components (or factors) that eventually results in a fibrin clot. Generally, the blood components participating in what has been referred to as the "coagulation cascade" are proenzymes or zymogens, i.e. enzymatically inactive proteins that are converted into an active form by the action of an activator. One of these coagulation factors is FVII.

FVII is a vitamin K-dependent plasma protein synthesized in the liver and secreted into the blood as a single-chain glycoprotein with a molecular weight of 53 kDa (Broze & Majerus, J Biol Chem 1980; 255:1242-1247). The FVII zymogen is converted into an activated form (FVIIa) by proteolytic cleavage at a single site, R152-I153, resulting in two chains linked by a single disulfide bridge. FVIIa in complex with tissue factor (TF), the FVIIa complex, is able to convert both factor IX and factor X into their activated forms, followed by reactions leading to rapid thrombin production and fibrin formation (Østerud & Rapaport, Proc Natl Acad Sci USA 1977; 74:5260-5264).

FVII undergoes post-translational modifications, including vitamin K-dependent carboxylation resulting in ten γ-carboxyglutamic acid residues in the N-terminal region of the molecule. Thus, residue number 6, 7, 14, 16, 19, 20, 25, 26, 29 and 35 shown in SEQ ID NO:1 are γ-carboxyglutamic acids residues in the Gla domain important for FVII activity. Other post-translational modifications include sugar moiety attachment at two naturally occurring N-glycosylation sites at position 145 and 322, respectively, and at two naturally occurring O-glycosylation sites at position 52 and 60, respectively.

The gene coding for human FVII (hFVII) has been mapped to chromosome 13 at q34-qter 9 (de Grouchy et al., Hum Genet 1984; 66:230-233). It contains nine exons and spans 12.8 Kb (O'Hara et al., Proc Natl Acad Sci USA 1987; 84:5158-5162). The gene organisation and protein structure of FVII are similar to those of other vitamin K-dependent procoagulant proteins, with exons la and 1b encoding for signal sequence; exon 2 the propeptide and Gla domain; exon 3 a short hydrophobic region; exons 4 and 5 the epidermal growth factor-like domains; and exon 6 through 8 the serine protease catalytic domain (Yoshitake et al., Biochemistry 1985; 24: 3736-3750).

Reports exist on experimental three-dimensional structures of hFVIIa (Pike et al., PNAS USA 1999; 96:8925-30 and Kemball-Cook et al., J Struct Biol 1999; 127:213-223), of hFVIIa in complex with soluble tissue factor using X-ray crystallographic methods (Banner et al., Nature 1996; 380:41 and Zhang et al., J Mol Biol 1999; 285: 2089), and of smaller fragments of hFVII (Muranyi et al., Biochemistry 1998; 37:10605 and Kao et al., Biochemistry 1999; 38:7097).

Some protein-engineered variants of FVII have been reported (Dickinson & Ruf, J Biol Chem 1997;272:19875-19879; Kemball-Cook et al., J Biol Chem 1998; 273:8516-8521; Bharadwaj et al., J Biol Chem 1996; 271:30685-30691; Ruf et al., Biochemistry 1999; 38:1957-1966, US 5,560,580; US 5,288,629; WO 01/83725; WO 02/22776; WO 02/077218; WO 03/027147; WO 02/38162; WO 03/037932; WO 99/20767; WO 00/66753 and WO 01/58935).

Reports exist on expression of FVII in BHK or other mammalian cells (WO 92/15686, WO 91/11514 and WO 88/10295) and co-expression of FVII and kex2 endoprotease in eukaryotic cells (WO 00/28065).

Commercial preparations of human recombinant FVIIa are sold as NovoSeven®. NovoSeven® is indicated for the treatment of bleeding episodes in hemophilia A or B patients. NovoSeven® is the only rFVIIa for effective and reliable treatment of bleeding episodes available on the market.

An inactive form of FVII in which arginine 152 and/or isoleucine 153 is/are modified has been reported in WO 91/11514. These amino acids are located at the activation site. WO 96/12800 describes inactivation of FVIIa by a serine proteinase inhibitor. Inactivation by carbamylation of FVIIa at the α-amino acid group I153 has been described by Petersen et al., EurJ Biochem 1999;261:124-129. The inactivated form is capable of competing with wild-type FVII or FVIIa for binding to TF and inhibiting clotting activity. The inactivated form of FVIIa is suggested to be used for treatment of patients being in hypercoagulable states, such as patients with sepsis, in risk of myocardial infarction or of thrombotic stroke.

WO 98/32466 suggests that FVII, among many other proteins, may be PEGylated but does not contain any further information in this respect.

WO 01/58935 discloses a new strategy for developing FVII or FVIIa molecules having *inter alia* an increased half-life.

A circulating rFVIIa half-life of 2.3 hours was reported in "Summary Basis for Approval for NovoSeven®", FDA reference number 96-0597. Relatively high doses and frequent administration are necessary to reach and sustain the desired therapeutic or prophylactic effect. As a consequence adequate dose regulation is difficult to obtain and the need of frequent intravenous administrations imposes restrictions on the patient's way of living.

rFVIIa treatment could be rendered more efficient if a FVIIa form could be used which is engineered in such way that its binding to TF is improved. Without being limited to a specific theory, such a FVIIa variant could improve treatment by the following mechanism: A modified FVIIa molecule with increased affinity for TF would enable a more efficient treatment of haemorrhage due to its ability to replace inactive FVII or inactivated FVIIa on TF hereby mediating a stronger amplification of the coagulation pathway and hence speed up the clot formation process and possibly even mediate formation of a stronger clot. Thus, such an improved FVIIa would be more efficient in stopping uncontrolled bleedings, for example in trauma patients.

This increased efficiency will be localized to places of tissue damage since this is the only place where cells (endothelial cells) bearing active TF are present. Thus, in addition to increased efficiency, a modified FVIIa with increased affinity for TF will constitute a safer procoagulant treatment due to the localization of the activity to sites of tissue injury, i.e. to the cells that are exposed from the endothelium, i.e. at sites where increased procoagulant activity is desirable.

Accordingly, the main object of the present invention is to provide FVII/FVIIa variants with an increased clotting efficiency, such as an increased clotting activity (reduced clotting time) and/or an ability to generate stronger clots. The variants may be further engineered to obtain an increased phospholipid membrane binding affinity. Such variants will increase the efficiency of FVIIa even further since such a molecule might target the TF present on platelets through their fusion with the so-called microparticles budded from e.g. TF- producing monocytes. This targeting will co-localize the increase in Factor X generation with the remainder of the clotting cascade i.e. at the site of thrombin and fibrin formation.

Another problem in current rFVIIa treatment is the relative instability of the molecule with respect to proteolytic degradation. Proteolytic degradation is a major obstacle for obtaining a preparation in solution as opposed to a lyophilized product. The advantage of obtaining a stable soluble preparation lies in easier handling for the patient, and, in the case of emergencies, quicker action, which potentially can become life saving. Attempts to prevent proteolytic degradation by site directed mutagenesis at major proteolytic sites have been disclosed in WO88/10295. Another attempt to prepare stablized liquid formulations of FVII/FVIIa is described in WO 03/055512.

Thus, a further object of the present invention is to provide FVII/FVIIa variants which, in addition to the above-mentioned improved properties, are more stable towards proteolytic degradation, i.e. possess reduced sensitivity to proteolytic degration.

WO02/38162 relates to modification of FVIIa to increase amidolytic activity in the absence of tissue factor and affinity for tissue factor when compared to the native factor Vila, while not substantially altering proteolytic activity when bound to tissue factor.

WO01/589 relates to FVII or FVIIa polypeptide conjugates comprising at least one non-polypeptide moiety covalently attached to a polypeptide, wherein the amino acid sequence of the polypeptide differs from that of wild-type FVII or FVIIa in that at least one amino acid residue comprising an attachment group for said non-polypeptide moiety has been introduced or removed.

Cheung Wing-Fai et al. (1995), Thrombosis Research, 80, 419-427, is concerned with the localisation of a metal-dependent epitope in FVII.

Dickinson et al. (1996), Proc Natl Acad Sci USA. 93, 14379-14384, reports a study concerned with the identification of surface residues involved in tissue factor binding and catalytic function of FVII. Dickinson *et al.* report that Gln-64, Ile-69, Phe-71, Arg-79, Arg-277, Met-306 and Asp-30 have an energetic influence.

Dickinson et al. (1997) Journal of Biological Chemistry, 272, 19875-19879, reports a study of affinities for zymogen and enzyme species of wild-type FVII and of mutants at protease domain residues that contact TF. The authors conclude that TF binding is not influenced by zymogen activation, and that there is bidirectional conformational interdependence between residue Met306 and the active site of FVII.

Petrovan et al., (2001) J Biol Chem. 276, 6616-6620, reports that residue Met 156 contributes to the labile enzyme conformation of coagulation factor VIIa.

Sridhara et al., (1996), Am J Hematol., 53, 66-71, is a study of the effect of mutations R152Q and R79Q in an ELISA assay which concludes that both the activation state of factor VII and the mutation of amino-acid residues within the first epidermal growth factor-like domain may alter the affinity of factor VII for tissue factor.

WO00/66753 relates to the membrane binding affinity of vitamin K-dependent polypeptides including FVII. Amino acid substitutions at amino acid 5, 9, 11, 12, 29, 33, 34, 35, or 36 are mentioned.

US 5,580,560 relates to modification of factor VII/VIIa for stabilization against proteolytic cleavage.

A molecule with a longer circulation half-life would decrease the number of necessary administrations. Given the association of current FVIIa product with frequent injections, and the potential for obtaining more optimal therapeutic FVIIa levels with concomitant enhanced therapeutic effect, there is a clear need for FVII or FVIIa molecules with an increased circulating half-life. One way to increase the circulation half-life of a protein is to ensure that renal clearance of the protein is reduced. This may be achieved by conjugating the protein to a chemical moiety which is capable of conferring reduced renal clearance to the protein. Furthermore, attachment of a chemical moiety to the protein or substitution of amino acids exposed to proteolysis may effectively block a proteolytic enzyme from contact leading to proteolytic degradation of the protein. Polyethylene glycol (PEG) is one such chemical moiety that has been used in the preparation of therapeutic protein products.

Thus, a still further objective of the present invention is to provide FVII/FVIIa variants which, in addition to the above-mentioned improved properties, have an increased functional *in vivo* half-life and/or an increased serum half-life.

The above-mentioned objectives are met by the improved FVII/FVIIa variants disclosed herein.

### BRIEF DISCLOSURE OF THE INVENTION

The present invention provides improved recombinant FVII or FVIIa variants comprising the substitution L65Q. This amino acid substitution in the TF binding site of the FVII molecule results in an improved clotting activity. The variants of the invention differ from human Factor VII or human Factor VIIa (SEQ ID NO:1) in no more than 15 amino acid residues.

In interesting embodiments, the FVII or FVIIa variant has been further modified so that the resulting variant has an enhanced phospholipid membrane binding affinity, increased functional *in vivo* half-life and/or increased plasma half-life. In still other embodiments, the variant has been further modified so as to possess increased bioavailability and/or reduced sensitivity to proteolytic degradation. Consequently, medical treatment with such a variant offers a number of advantages over the currently available rFVIIa compound, such as lower dosage, faster action in uncontrolled bleedings and, optionally, longer duration between injections.

In one aspect the invention relates to a Factor VII (FVII) or Factor VIIa (FVIIa) polypeptide variant having an amino acid sequence comprising 1-15 amino acid modifications relative to human Factor VII (HFVII) or human Factor VIIa (hFVIIa) having the amino acid sequence shown in SEQ ID NO:1, wherein said variant sequence comprises the substitution L65Q
with the proviso that said variant is not
[K32E+D33N+A34T+K38T+L39E]hFVII or
[A 1Y+K32E+D33N+A34T+K38T+L39E]hFVII or
[A1Y+A3S+F4GK+K32E+D33N+A34T+K38T+L39E]hFVII or
[A1Y+L8F+R9V+P10Q+K32E+D33N+A34T+K38T+L39E]hFVII or
[A1Y+A3S+F4GK+L8F+R9V+P10Q+K32E+D33N+A34T+K38T+L39E]hFVII or
[A3S+F4GK+K32E+D33N+A34T+K38T+L39E]hFVII or
[A3S+F4GK+L8F+R9V+P10Q+K32E+D33N+A34T+K38T+L39E]hFVII or
[L8F+R9V+P10Q+K32E+D33N+A34T+K38T+L39E]hFVII or
[I42N]hFVII/hFVIIa or [I42S]hFVII/hFVIIa or [I42A]hFVII/hFVIIa or
[I42Q]hFVII/hFVIIa.

In a second aspect the invention relates to a Factor VIIa (FVIIa) polypeptide variant having an amino acid sequence comprising 1-15 amino acid modifications relative to human Factor VIIa (hFVIIa) having the amino acid sequence shown in SEQ ID NO:1, wherein said variant sequence comprises the substitution. The FVII variants comprising the L39E mutation disclosed by Cheung and Stafford Throm Res 1995;79;199-206 are not within the scope of the present application. Likewise, the FVII/FVIIa variants comprising the I42N/S/A/Q mutations disclosed in US 5,580,560 are not within the scope of the present application.

Another aspect of the invention relates to a nucleotide sequence encoding the variant of the invention.

In a further aspect the invention relates to an expression vector comprising the nucleotide sequence of the invention.

In a still further aspect the invention relates to a host cell comprising the nucleotide sequence of the invention or the expression vector of the invention.

In an even further aspect the invention relates to a pharmaceutical composition comprising variant of the invention, and a pharmaceutical acceptable carrier or excipient.

Still another aspect of the invention relates to the variant of the invention, or the pharmaceutical composition of the invention, for use as a medicament.

Further aspects of the present invention will be apparent from the below description as well as from the appended claims.

### BRIEF DESCRIPTION OF THE DRAWINGS

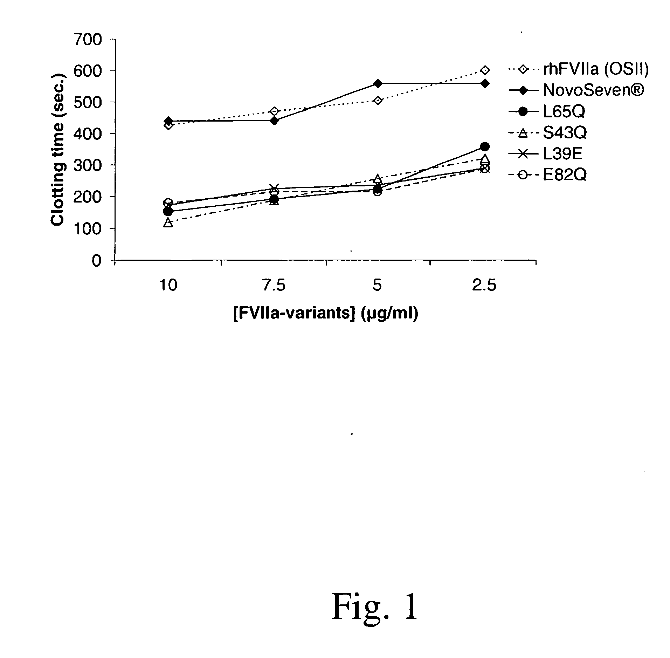

Fig. 1 shows the clotting time vs. concentration for [L65Q]rhFVIIa, [S43Q]rhFVIIa, [L39E]rhFVIIa and [E82Q]rhFVIIa when assayed in the "Whole Blood Assay". For comparison, the results for rhFVIIa (OSII-activated) and NovoSeven® are included. 0 rhFVIIa (OSII-activated); ◆ NovoSeven®; ●[L65Q]rhFVIIa; Δ [S43Q]rhFVIIa; x [L39E]rhFVIIa; O [E82Q]rhFVIIa.

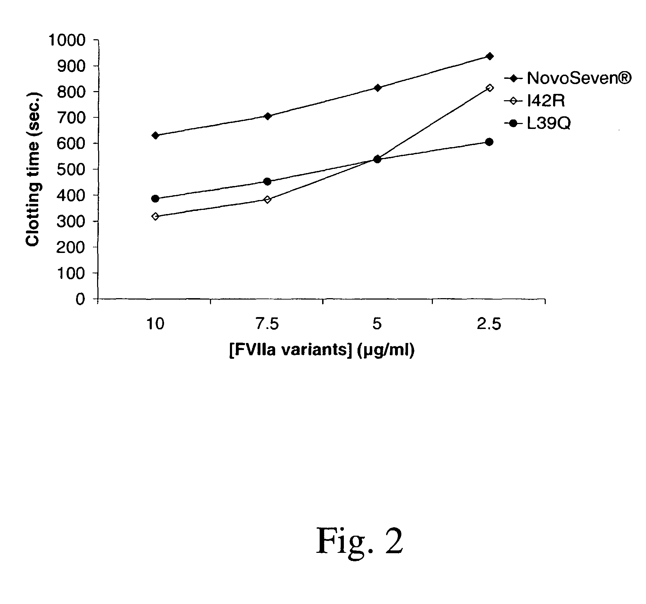

Fig. 2 shows the clotting time vs. concentration for [I42R]rhFVIIa and [L39Q]rhFVIIa when assayed in the "Whole Blood Assay". For comparison, the result for NovoSeven® is included. ◆ NovoSeven®; 0 [I42R]rhFVIIa; ● [L39Q]rhFVIIa.

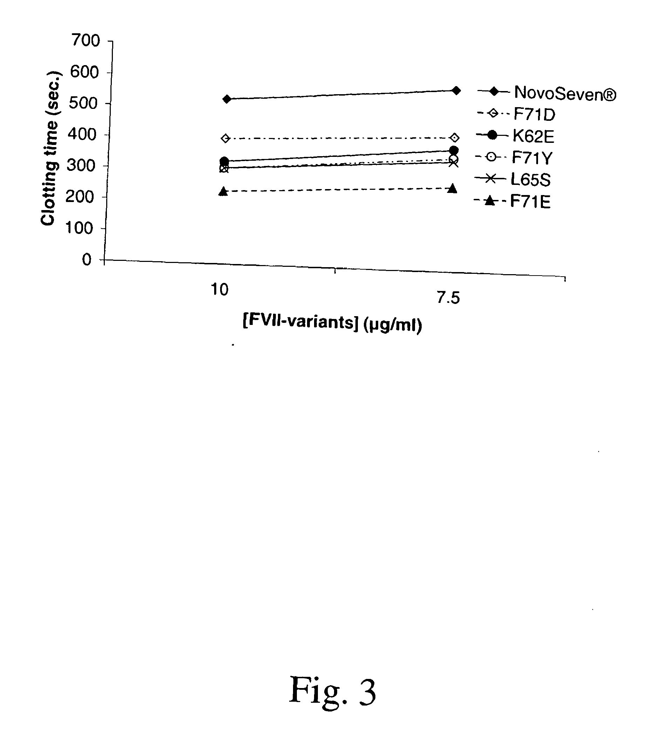

Fig. 3 shows the clotting time vs. concentration for [F71D]rhFVIIa, [K62E]rhFVIIa, [F71Y]rhFVIIa, [L65S]rhFVIIa and [F71E]rhFVIIa when assayed in the "Whole Blood Assay". For comparison, the result for NovoSeven® is included.◆ NovoSeven®; ◊ [F71D]rhFVIIa; ● [K62E]rhFVIIa; o [F71Y]rhFVIIa; x [L65S]rhFVIIa; ▲ [F71E]rhFVIIa.

### DETAILED DISCLOSURE OF THE INVENTION

### Definitions

In the context of the present application and invention the following definitions apply:

The term "conjugate" (or interchangeably "conjugated polypeptide variant") is intended to indicate a heterogeneous (in the sense of composite or chimeric) molecule formed by the covalent attachment of one or more polypeptide(s) to one or more non-polypeptide moieties such as polymer molecules, lipophilic compounds, sugar moieties or organic derivatizing agents. Preferably, the conjugate is soluble at relevant concentrations and conditions, i.e. soluble in physiological fluids such as blood. Examples of conjugated polypeptide variants of the invention include glycosylated and/or PEGylated polypeptides.

The term "covalent attachment" or "covalently attached" means that the polypeptide variant and the non-polypeptide moiety are either directly covalently joined to one another, or else are indirectly covalently joined to one another through an intervening moiety or moieties, such as a bridge, spacer, or linkage moiety or moieties.

When used herein, the term "non-polypeptide moiety" means a molecule that is capable of conjugating to an attachment group of the polypeptide variant of the invention. Preferred examples of such molecules include polymer molecules, sugar moieties, lipophilic compounds, or organic derivatizing agents, in particular sugar moieties. When used in the context of a polypeptide variant of the invention it will be understood that the non-polypeptide moiety is linked to the polypeptide part of the polypeptide variant through an attachment group of the polypeptide variant. As explained above, the non-polypeptide moiety may be directly covalently joined to the attachment group or it may be indirectly covalently joined to the attachment group through an intervening moiety or moieties, such as a bridge, spacer, or linkage moiety or moieties.

A "polymer molecule" is a molecule formed by covalent linkage of two or more monomers, wherein none of the monomers is an amino acid residue. The term "polymer" may be used interchangeably with the term "polymer molecule". The term is also intended to cover carbohydrate molecules attached *by in vitro* glycosylation, i.e. a synthetic glycosylation performed *in vitro* normally involving covalently linking a carbohydrate molecule to an attachment group of the polypeptide variant, optionally using a cross-linking agent. *In vitro* glycosylation is discussed in detail further below.

The term "sugar moiety" is intended to indicate a carbohydrate-containing molecule comprising one or more monosaccharide residues, capable of being attached to the polypeptide variant (to produce a polypeptide variant conjugate in the form of a glycosylated polypeptide variant) by way of *in vivo* glycosylation. The term *"in vivo* glycosylation" is intended to mean any attachment of a sugar moiety occurring *in vivo,* i.e. during posttranslational processing in a glycosylating cell used for expression of the polypeptide variant, e.g. by way of N-linked or O-linked glycosylation. The exact oligosaccharide structure depends, to a large extent, on the glycosylating organism in question.

An "N-glycosylation site" has the sequence N-X-S/T/C, wherein X is any amino acid residue except proline, N is asparagine and S/T/C is either serine, threonine or cysteine, preferably serine or threonine, and most preferably threonine. Preferably, the amino acid residue in position +3 relative to the asparagine residue is not a proline residue.

An "O-glycosylation site" is the OH-group of a serine or threonine residue.

The term "attachment group" is intended to indicate a functional group of the polypeptide variant, in particular of an amino acid residue thereof or a carbohydrate moiety, capable of attaching a non-polypeptide moiety such as a polymer molecule, a lipophilic molecule, a sugar moiety or an organic derivatizing agent. Useful attachment groups and their matching non-polypeptide moieties are apparent from the table below.

| Attachment group | Amino acid | Examples of non-polypeptide moiety | Conjugation method/-Activated PEG | Reference |
|---|---|---|---|---|
| -NH₂ | N-terminal, Lys | Polymer, e.g. PEG, with amide or imine group | mPEG-SPA Tresylated mPEG | Shearwater Inc. Delgado et al., *Critical Reviews in Therapeutic Drug Carrier Systems* 9(3,4):249-304 (1992) |
| -COOH | C-terminal, Asp, Glu | Polymer, e.g. PEG, with ester or amide group Carbohydrate moiety | mPEG-Hz *In vitro* coupling | Shearwater Inc. |
| -SH | Cys | Polymer, e.g. PEG, with disulfide, maleimide or vinyl sulfone group Carbohydrate moiety | PEG-vinylsul- phone PEG-maleimide *In vitro* coupling | Shearwater Inc. Delgado et al, critical reviews in Therapeutic Drug Carrier Systems 9(3,4):249-304 (1992) |
| -OH | Ser, Thr, Lys, OH- | Sugar moiety PEG with ester, ether, carbamate, carbonate | *In vivo* O-linked glycosylation | |
| -CONH₂ | Asn as part of an N-glycosylation site | Sugar moiety Polymer, e.g. PEG | *In vivo N-* glycosylation | |
| Aromatic residue | Phe, Tyr, Trp | Carbohydrate moiety | *In vitro coupling* | |
| -CONH₂ | Gln | Carbohydrate moiety | *In vitro* coupling | Yan and Wold, Biochemistry, 1984, Jul 31; 23(16): 3759-65 |
| Aldehyde Ketone | Oxidized oligo-saccharide | Polymer, e.g. PEG, PEG-hydrazide | PEGylation | Andresz et al., 1978, Makromol. Chem. 179:301, WO 92/16555, WO 00/23114 |
| Guanidino | Arg | Carbohydrate moiety | *In vitro* coupling | Lundblad and Noyes, Chimical Reagents for Protein Modification, CRC Press Inc., Florida, USA |
| Imidazole ring | His | Carbohydrate moiety | *In vitro* coupling | As for guanidine |

For *in vivo* N-glycosylation, the term "attachment group" is used in an unconventional way to indicate the amino acid residues constituting a N-glycosylation site (with the sequence N-X-S/T/C, wherein X is any amino acid residue except proline, N is asparagine and S/T/C is either serine, threonine or cysteine, preferably serine or threonine, and most preferably threonine). Although the asparagine residue of the N-glycosylation site is the one to which the sugar moiety is attached during glycosylation, such attachment cannot be achieved unless the other amino acid residues of the N-glycosylation site are present.

Accordingly, when the non-polypeptide moiety is a sugar moiety and the conjugation is to be achieved by *in vivo* N-glycosylation, the term "amino acid residue comprising an attachment group for the non-polypeptide moiety" as used in connection with alterations of the amino acid sequence of the polypeptide variant is to be understood as meaning that one or more amino acid residues constituting an *in vivo* N-glycosylation site are to be altered in such a manner that either a functional *in vivo* N-glycosylation site is introduced into the amino acid sequence or removed from said sequence.

In the present application, amino acid names and atom names (e.g. CA, CB, CD, CG, SG, NZ, N, O, C, etc) are used as defined by the Protein DataBank (PDB) (www.pdb.org) based on the IUPAC nomenclature (IUPAC Nomenclature and Symbolism for Amino Acids and Peptides (residue names, atom names, etc.), Eur. J. Biochem., 138, 9-37 (1984) together with their corrections in Eur. J. Biochem., 152, 1 (1985)).

The term "amino acid residue" is intended to indicate an amino acid residue contained in the group consisting of alanine (Ala or A), cysteine (Cys or C), aspartic acid (Asp or D), glutamic acid (Glu or E), phenylalanine (Phe or F), glycine (Gly or G), histidine (His or H), isoleucine (Ile or I), lysine (Lys or K), leucine (Leu or L), methionine (Met or M), asparagine (Asn or N), proline (Pro or P), glutamine (Gln or Q), arginine (Arg or R), serine (Ser or S), threonine (Thr or T), valine (Val or V), tryptophan (Trp or W), and tyrosine (Tyr or Y) residues.

The terminology used for identifying amino acid positions is illustrated as follows: G124 indicates that position 124 is occupied by a glycine residue in the amino acid sequence shown in SEQ NO:1. G124R indicates that the glycine residue of position 124 has been substituted with an arginine residue. Alternative substitutions are indicated with a "/", e.g. N145S/T means an amino acid sequence in which asparagine in position 145 is substituted with either serine or threonine. Multiple substitutions are indicated with a "+", e.g. K143N+N145S/T means an amino acid sequence which comprises a substitution of the lysine residue in position 143 with an asparagine residue and a substitution of the asparagine residue in position 145 with a serine or a threonine residue. Insertion of an additional amino acid residue, such as insertion of an alanine residue after G124 is indicated by G124GA. Insertion of two additional alanine residues after G124 is indicated by G124GAA, etc. When used herein, the term "inserted in position X" or "inserted at position X" means that the amino acid residue(s) is (are) inserted between amino acid residue X and X+1. A deletion of an amino acid residue is indicated by an asterix. For example, deletion of the glycine residue in position 124 is indicated by G124*.

Unless otherwise indicated, the numbering of amino acid residues made herein is made relative to the amino acid sequence of human wild-type FVII/FVIIa (SEQ ID NO: 1).

The term "differs from" as used in connection with specific mutations is intended to allow for additional differences being present apart from the specified amino acid difference. For instance, in addition to the specified substitutions in positions 39, 42, 43, 62, 65, 71, 82 and/or 275, the FVII or FVIIa polypeptide variant may comprise other substitutions. Examples of such additional modifications or differences may include truncation of the N- and/or C-terminus by one or more amino acid residues (e.g. by 1-10 amino acid residues), or addition of one or more extra residues at the N- and/or C-terminus, e.g. addition of a methionine residue at the N-terminus as well as "conservative amino acid substitutions", i.e. substitutions performed within groups of amino acids with similar characteristics, e.g. small amino acids, acidic amino acids, polar amino acids, basic amino acids, hydrophobic amino acids and aromatic amino acids.

Examples of such conservative substitutions are shown in the below table.

| | | | | |
|---|---|---|---|---|
| 1 | Alanine (A) | Glycine (G) | Serine (S) | Threonine (T) |
| 2 | Aspartic acid (D) | Glutamic acid (E) | | |
| 3 | Asparagine (N) | Glutamine (Q) | | |
| 4 | Arginine (R) | Histidine (H) | Lysine (K) | |
| 5 | Isoleucine (I) | Leucine (L) | Methionine (M) | Valine (V) |
| 6 | Phenylalanine (F) | Tyrosine (Y) | Tryptophan (W) | |

Still other examples of additional modifications include modifications giving rise to an increased functional *in vivo* half-life or an increased serum half-life. Specific examples of such modifications are discussed further below. Moreover, the polypeptide variant of the invention may contain additional modifications giving rise to an enhanced phospholipid membrane binding affinity. Specific examples are given below.

The term "nucleotide sequence" is intended to indicate a consecutive stretch of two or more nucleotide molecules. The nucleotide sequence may be of genomic, cDNA, RNA, semisynthetic, synthetic origin, or any combinations thereof.

The term "polymerase chain reaction" or "PCR" generally refers to a method for amplification of a desired nucleotide sequence *in vitro,* as described, for example, in US 4,683,195. In general, the PCR method involves repeated cycles of primer extension synthesis, using oligonucleotide primers capable of hybridising preferentially to a template nucleic acid.

The term "vector" refers to a plasmid or other nucleotide sequences that are capable of replicating within a host cell or being integrated into the host cell genome, and as such, are useful for performing different functions in conjunction with compatible host cells (a vector-host system): to facilitate the cloning of the nucleotide sequence, i.e. to produce usable quantities of the sequence, to direct the expression of the gene product encoded by the sequence and to integrate the nucleotide sequence into the genome of the host cell. The vector will contain different components depending upon the function it is to perform.

"Cell", "host cell", "cell line" and "cell culture" are used interchangeably herein and all such terms should be understood to include progeny resulting from growth or culturing of a cell.

"Transformation" and "transfection" are used interchangeably to refer to the process of introducing DNA into a cell.

"Operably linked" refers to the covalent joining of two or more nucleotide sequences, by means of enzymatic ligation or otherwise, in a configuration relative to one another such that the normal function of the sequences can be performed. For example, the nucleotide sequence encoding a presequence or secretory leader is operably linked to a nucleotide sequence coding for a polypeptide if it is expressed as a preprotein that participates in the secretion of the polypeptide: a promoter or enhancer is operably linked to a coding sequence if it affects the transcription of the sequence; a ribosome binding site is operably linked to a coding sequence if it is positioned so as to facilitate translation. Generally, "operably linked" means that the nucleotide sequences being linked are contiguous and, in the case of a secretory leader, contiguous and in reading phase. Linking is accomplished by ligation at convenient restriction sites. If such sites do not exist, then synthetic oligonucleotide adaptors or linkers are used, in conjunction with standard recombinant DNA methods.

In the context of the present invention the terms "modification" or "amino acid modification" is intended to cover replacement of an amino acid side chain, substitution of an amino acid residue, deletion of an amino acid residue and/or insertion of an amino acid residue of interest.

The terms "mutation" and "substitution" are used interchangeably herein.

The term "introduce" refers to introduction of an amino acid residue by substitution of an existing amino acid residue, or alternatively by insertion of an additional amino acid residue.

The term "remove" refers to removal of an amino acid residue by substitution of the amino acid residue to be removed by another amino acid residue, or alternatively by deletion (without substitution) of the amino acid residue to be removed.

The term "FVII" or "FVII polypeptide" refers to a FVII molecule provided in single chain form. One example of a FVII polypeptide is the wild-type human FVII (hFVII) having the amino acid sequence shown in SEQ ID NO:1. It should be understood, however, that the term "FVII polypeptide" also covers hFVII-like molecules, such as fragments or variants of SEQ ID NO:1, in particular variants where the sequence comprises at least one, such as 1-15, preferably 1-10, amino acid modifications as compared to SEQ ID NO:1

The term "FVIIa" or "FVIIa polypeptide" refers to a FVIIa molecule provided in its activated two-chain form. When the amino acid sequence of SEQ ID NO: is used to describe the amino acid sequence of FVIIa it will be understood that the peptide bond between R152 and I153 of the single-chain form has been cleaved, and that one of the chains comprises amino acid residues 1-152, the other chain amino acid residues 153-406..

The terms "rFVII" and "rFVIIa" refer to FVII and FVIIa molecules produced by recombinant techniques, respectively.

The terms "hFVII" and "hFVIIa" refer to wild-type human FVII and FVIIa, respectively, having the amino acid sequence shown in SEQ ID NO:1.

The terms "rhFVII" and "rhFVIIa" refer to human wild-type FVII and FVIIa, having the amino acid sequence shown in SEQ ID NO:1, produced by recombinant means. An example of rhFVIIa is NovoSeven®.

When used herein, the term "Gla domain" is intended to cover amino acid residues no. 1 to 45 of SEQ ID NO:1. Accordingly, the term "position located outside the Gla domain" covers amino acid residue no. 46-406 of SEQ ID NO:1.

The abbreviations "FX", "TF" and "TFPI" mean Factor X, Tissue Factor and Tissue Factor Pathway Inhibitor, respectively.

The term "protease domain" is used about residues 153-406 counted from the N-terminus.

The term "catalytic site" is used to mean the catalytic triad consisting of S344, D242 and H193 of the FVII/FVIIa molecule.

The term "parent" is intended to indicate the molecule to be modified/improved in accordance with the present invention. Although the parent polypeptide to be modified by the present invention may be any FVII or FVIIa polypeptide, and thus be derived from any origin, e.g. a non-human mammalian origin, it is preferred that the parent polypeptide is hFVII or hFVIIa.

A "variant" is a polypeptide, which differs in one or more amino acid residues from its parent polypeptide, normally in 1-15 amino acid residues (e.g. in 1,2, 3,4, 5, 6, 7, 8, 9, 10, 11, 12, 13, 14 or 15 amino acid residues), such as in 1-10 amino acid residues, e.g. in 1-8, 1-6, 1-5 or 1-3 amino acid residues. Normally, the parent polypeptide is HFVII or hFVIIa. Thus, a "variant" typically contains 1-15 amino acid modifications (for example 1, 2, 3, 4, 5, 6, 7, 8, 9, 10, 11, 12, 13, 14 or 15 amino acid modifications), such as 1-10 amino acid modifications, e.g. 1-8, 1-6, 1-5 or 1-3 amino acid modifications relative to the parent polypeptide. As explained above, the parent polypeptide is normally hFVII or hFVIIa. It will be understood that a polypeptide variant according to the present invention will differ from the SEQ ID NO:1 in at least one of the following positions: L39, I42, S43, K62, L65, F71, E82 and/or F275.

In the present context, the term "modification" encompasses insertions, deletions, substitutions and combinations thereof. It will be understood that a polypeptide variant according to the present invention will be modified in at least one position relative to the parent polypeptide.

The term "amidolytic activity" is intended to mean the activity measured in the "Amidolytic Assay" described herein. In order to exhibit "amidolytic activity" a variant of the invention, in its activated form, should have at least 10% of the amidolytic acitivty of rhFVIIa when assayed in the "Amidolytic Assay" described herein. In a preferred embodiment of the invention the variant, in its activated form, has at least 20% of the amidolytic activity of rhFVIIa, such as at least 30%, e.g. at least 40%, more preferably at least 50%, such as at least 60%, e.g. at least 70%, even more preferably at least 80%, such as at least 90% of the amidolytic activity of rhFVIIa when assayed in the "Amidolytic Assay" described herein. In an interesting embodiment the variant, in its activated form, has substantially the same amidolytic activity as rhFVIIa, such as an amidolytic activity of 75-125% of the amidolytic acitivity of rhFVIIa.

The term "clotting activity" is used to mean the activity measured in the "Whole Blood Assay" described herein. It will be understood that the activity measured in the "Whole Blood Assay" is the time needed to obtain clot formation. Thus, a lower clotting time corresponds to a higher clotting activity.

The term "increased clotting activity" is used to indicate that the clotting time of the polypeptide variant is statistically significantly decreased relative to that generated by rhFVIIa as determined under comparable conditions and when measured in the "Whole Blood Assay" described herein.

The term "immunogenicity" as used in connection with a given substance is intended to indicate the ability of the substance to induce a response from the immune system. The immune response may be a cell or antibody mediated response (see, e.g., Roitt: Essential Immunology (8^{th} Edition, Blackwell) for further definition of immunogenicity). Normally, reduced antibody reactivity will be an indication of reduced immunogenicity. The reduced immunogenicity may be determined by use of any suitable method known in the art, e.g. *in vivo* or *in vitro.*

The term "functional *in vivo* half-life" is used in its normal meaning, i.e. the time at which 50% of the biological activity of the polypeptide variant is still present in the body/target organ, or the time at which the amidolytic or clotting activity of the polypeptide variant is 50% of the initial value.

As an alternative to determining functional *in vivo* half-life, "serum half-life" may be determined, i.e. the time at which 50% of the polypeptide variant circulates in the plasma or bloodstream prior to being cleared. Determination of serum half-life is often more simple than determining the functional *in vivo* half-life and the magnitude of serum half-life is usually a good indication of the magnitude of functional *in vivo* half-life. Alternatively terms to serum half-life include "plasma half-life", "circulating half-life", "serum clearance", "plasma clearance" and "clearance half-life". The polypeptide variant is cleared by the action of one or more of the reticuloendothelial systems (RES), kidney, spleen or liver, by tissue factor, SEC receptor or other receptor mediated elimination, or by specific or unspecific proteolysis. Normally, clearance depends on size (relative to the cutoff for glomerular filtration), charge, attached carbohydrate chains, and the presence of cellular receptors for the protein. The functionality to be retained is normally selected from procoagulant, proteolytic or receptor binding activity. The functional *in vivo* half-life and the serum half-life may be determined by any suitable method known in the art.

The term "increased" as used about the functional *in vivo* half-life or serum half-life is used to indicate that the relevant half-life of the polypeptide variant is statistically significantly increased relative to that of a reference molecule, such as a hFVIIa or rhFVIIa (e.g. NovoSeven®) as determined under comparable conditions (typically determined in an experimental animal, such as rats, rabbits or pigs).

The term "AUCᵢᵥ" or "Area Under the Curve when administered intravenously" is used in its normal meaning, i.e. as the area under the activity in serum-time curve, where the polypeptide variant has been administered intravenously, in particular when administered intravenously in rats. Typically, the activity measured is the "clotting activity" as defined hereinbefore. Once the experimental activity-time points have been determined, the AUCᵢᵥ may conveniently be calculated by a computer program, such as GraphPad Prism 3.01.

It will be understood that in order to make a direct comparison between the AUCᵢᵥ-values of different molecules (e.g. between the variants of the invention and rhFVIIa the same amount of activity should be administered. Consequently, the AUCᵢᵥ-values are typically normalized (i.e. corrected for differences in the injected dose) and expressed as AUCᵢᵥ/dose administered.

The term "reduced sensitivity to proteolytic degradation" is primarily intended to mean that the polypeptide variant has reduced sensitivity to proteolytic degradation in comparison to hFVIIa or rhFVIIa (e.g. NovoSeven®) as determined under comparable conditions. Preferably, the proteolytic degradation is reduced by at least 10% (e.g. by 10-25% or by 10-50%), such as at least 25% (e.g. by 25-50%, by 25-75% or by 25-100%), more preferably by at least 35%, such as at least 50%, (e.g. by 50-75% or by 50-100%) even more preferably by at least 60%, such as by at least 75% (e.g.,by 75-100%) or even at least 90%. Most preferably, the proteolytic degradation is reduced by at least 99%.

The term "renal clearance" is used in its normal meaning to indicate any clearance taking place by the kidneys, e.g. by glomerular filtration, tubular excretion or degradation in the tubular cells. Renal clearance depends on physical characteristics of the polypeptide, including size (diameter), hydrodynamic volume, symmetry, shape/rigidity, and charge. Normally, a molecular weight of about 67 kDa is considered to be a cut-off-value for renal clearance. Renal clearance may be established by any suitable assay, e.g. an established *in vivo* assay. Typically, renal clearance is determined by administering a labelled (e.g. radiolabelled or fluorescence labelled) polypeptide to a patient and measuring the label activity in urine collected from the patient. Reduced renal clearance is determined relative to a corresponding reference polypeptide, e.g. human wild-type FVIIa, under comparable conditions. Preferably, the renal clearance rate of the polypeptide variant is reduced by at least 50%, preferably by at least 75%, and most preferably by at least 90% compared to hFVIIa or rhFVIIa (e.g. NovoSeven®).

The terms "at least 25% of its side chain exposed to the surface of the molecule" and "at least 50% of its side chain exposed to the surface of the molecule" are defined with reference to Example 1, where the calculations, etc. are described in detail.

It should be noted that when the terms "at least 25% of its side chain exposed to the surface of the molecule" and "at least 50% of its side chain exposed to the surface of the molecule" are used in connection with introduction of an *in vivo* N-glycosylation site these terms refer to the surface accessibility of the amino acid side chain in the position where the sugar moiety is actually attached. In many cases it will be necessary to introduce a serine or a threonine residue in position +2 relative to the asparagine residue to which the sugar moiety is actually attached (unless, of course, this position is already occupied by a serine or a threonine residue) and these positions, where the serine or threonine residues are introduced, are allowed to be buried, i.e. to have less than 25% or 50% of their side chains exposed to the surface of the molecule.

In the present description and claims, any reference to "a" component, e.g. in the context of a non-polypeptide moiety, an amino acid residue, a substitution, a buffer, etc., is intended to refer to one or more of such components, unless stated otherwise or unless it is clear from the particular context that this is not the case. For example, the expression "a component selected from the group consisting of A, B and C" is intended to include all combinations of A, B and C, i.e. A, B, C, A+B, A+C, B+C or A+B+C.

A polypeptide, nucleotide sequence or other component is "isolated" when it is partially or completely separated from components with which it is normally associated (other peptides, polypeptides, proteins (including complexes, e.g., polymerases and ribosomes which may accompany a native sequence), nucleic acids, cells, synthetic reagents, cellular contaminants, cellular components, etc.), e.g., such as from other components with which it is normally associated in the cell from which it was originally derived. A polypeptide, nucleotide sequence, or other component is isolated when it is partially or completely recovered or separated from other components of its natural environment such that it is the predominant species present in a composition, mixture, or collection of components (i.e., on a molar basis it is more abundant than any other individual species in the composition). In some instances, the preparation consists of more than about 60%, more than about 70% or more than about 75%, typically more than about 80%, or preferably more than about 90% of the isolated species.

The terms "tissue factor binding site", "active site region" and "ridge of the active site binding cleft" are defined with reference to Example 1, wherein the above-mentioned sites/regions are determined.

The term "hydrophobic amino acid residue" includes the following amino acid residues: Ile, Leu, Met, Val, Phe, Tyr and Trp.

The term "charged amino acid residue" encompasses the following amino acid residues: Lys, Arg, His, Asp and Glu.

The term "negatively charged amino acid residue" includes the following amino acid residues: Asp and Glu.

The term "positively charged amino acid residue" includes the following amino acid residues: Lys, Arg and His.

The term "polar amino acid residue" encompasses the following amino acid residues: Gly, Ser, Thr, Cys, Tyr, Asn and Gln.

The term "mammal" as used herein includes humans, non-human primates (e.g., baboons, orangutans, monkeys), mice, pigs, cows, goats, cats, rabbits, rats, guinea pigs, hamsters, horses, monkeys, sheep, or other non-human mammal.

The term "effective amount" means a dosage or amount sufficient to produce a desired result. The desired result may comprise an objective or subjective improvement in the recipient of the dosage or amount.

### Variants of the invention

In one aspect the present invention relates to a Factor VII (FVII) or Factor VIIa (FVIIa) polypeptide variant having an amino acid sequence comprising 1-15 amino acid modifications relative to human Factor VII (HFVII) or human Factor VIIa (hFVIIa) having the amino acid sequence shown in SEQ ID NO:1, wherein said variant sequence comprises the substitution L65Q,
with the proviso that said variant is not
[K32E+D33N+A34T+K38T+L39E]hFVII/hFVIIa or
[A1Y+K32E+D33N+A34T+K38T+L39E]hFVII/hFVIIa or
[A1Y+A3S+F4GK+K32E+D33N+A34T+K38T+L39E]hFVII/hFVIIa or
[A1Y+L8F+R9V+P10Q+K32E+D33N+A34T+K38T+L39E]hFVII/hFVIIa or
[A1Y+A3S+F4GK+L8F+R9V+P10Q+K32E+D33N+A34T+K38T+L39E]hFVII/hFVIIa or
[A3S+F4GK+K32E+D33N+A34T+K38T+L39E]hFVII/hFVIIa or
[A3S+F4GK+L8F+R9V+P10Q+K32E+D33N+A34T+K38T+L39E]hFVII/hFVIIa or
[LBF+R9V+P10Q+K32E+D33N+A34T+K38T+L39E]hFVII/FVIIa or
[142N]hFVU//hFVHa or [I42S]hFVII/hFVIIa or [I42A]hFVII/hFVIIa or
[I42Q]hFVII/hFVIIa.

In a further interesting embodiment of the invention, the FVII or FVIIa variant comprises the substitution L39E.

In a further interesting embodiment of the invention, the FVII or FVIIa variant comprises the substitution L39Q.

In a further interesting embodiment of the invention, the FVII or FVIIa variant comprises the substitution L39H.

In a further interesting embodiment of the invention, the FVII or FVIIa variant comprises the substitution I42R.

In a further interesting embodiment of the invention, the FVII or FVIIa variant comprises the substitution S43Q.

In a further interesting embodiment of the invention, the FVII or FVIIa variant comprises the substitution K62E.

In a further interesting embodiment of the invention, the FVII or FVIIa variant comprises the substitution K62R.

In all embodiments of the invention, the FVII or FVIIa variant comprises the substitution L65/Q.

In a further interesting embodiment of the invention, the FVII or FVIIa variant comprises the substitution L65S.

In a further interesting embodiment of the invention, the FVII or FVIIa variant comprises the substitution F71D.

In a further interesting embodiment of the invention, the FVII or FVIIa variant comprises the substitution F71Y.

In a further interesting embodiment of the invention, the FVII or FVIIa variant comprises the substitution F71E.

In a further interesting embodiment of the invention, the FVII or PVIIa variant comprises the substitution F71Q.

In a further interesting embodiment of the invention, the FVII or FVIIa variant comprises the substitution F7IN.

In a further interesting embodiment of the invention, the FVII or FVIIa variant comprises the substitution E82Q.

In a further interesting embodiment of the invention, the FVII or FVIIa variant comprises the substitution E82N.

In a further interesting embodiment of the invention, the FVII or FVIIa variant comprises the substitution F275H.

In a highly interesting embodiment of the invention, the FVII or FVIIa variant of the invention comprises a substitution selected from the group consisting of F71Y, K62E and S43Q, in particular selected from the group consisting of K62E and S43Q.

It will be understood that it may be advantageous to combine one or more of the above-mentioned substitutions. Accordingly, in a further interesting embodiment of the invention, the variant comprises at least two (such as two) substitutions in positions selected from the group consisting of L39,I142,S43, K62, L65, F71, E82 and F275, such as substitutions in positions selected from the group consisting of L39+I42, L39+S43, L39+K62, L39+L65, L39+F71, L39+E82, L39+F275, I42+S43, I42+K62, I42+L65, I42+F71, I42+F71, I42+E82, 142+F275, S43+K62, S43+L65, S43+F71, S43+E82, S43+F275, K62+L65, K62+F71, K62+E82, K62+F275, L65+F71, L65+E82, L65+F275, F71+E82, F71+F275 and E82+F275. According to this embodiment of the invention, it is preferred that the variant comprises at least two (such as two) substitutions in positions selected from the group consisting of L65+F71, L65+K62, L65+S43, F71+K62, F71+S43 and K62+S43, in particular at least two (such as two) substitutions in positions selected from the group consisting of L65+K62, L65+S43 and, K62+S43. More particularly, the variant of the invention may comprise at least two (such as two) substitutions selected from the group consisting of L39E, L39Q, L39H, I42R, S43Q, K62E, K62R, L65Q, L65S, F71D, F71Y, F71E, F71Q, F71N, E82Q, E82N and F275H, preferably at least two (such as two) substitutions selected from the group consisting of L65Q, F71Y, K62E and S43Q, more preferably at least two (such as two) substitutions selected from the group consisting of L65Q, K62E and S43Q. Specific examples include L65Q+F71 Y, L65Q+K62E, L65Q+S43Q, F71Y+K62E, F71Y+S43Q and K62E+S43Q, in particular L65Q+K62E, L65Q+S43Q and K62E+S43Q.

In a still further interesting embodiment of the invention, the variant comprises at least three (such as three) substitutions in positions selected from the group consisting of L39, I42, S43, K62, F71, E82 and F275, such as substitutions in positions selected from the group consisting of L39+I42+S43, L39+I42+K62, L39+I42+L65, L39+I42+F71, L39+I42+E82, L39+I42+F275, L39+543+K62, L39+S43+L65, L39+S43+F71, L39+K62+E82, L39+S43+F275, L39+K62+L65, L39+K62+F71, L39+K62+E82, L39+K62+F275, L39+L65+F71, L39+L65+E82, L39+L65+F275, L39+F71+E82, L39+F71+F275, L39+E82+F275, I42+S43+K62, I42+S43+L65, I42+S43+F71, I42+S43+E82, I42+S43+F275, I42+K62+L65, I42+K62+F71, I42+K62+E82, I42+K62+F275, I42+L65+F71, I42+L65+E82, I42+L65+F275,142+F71+E82, I42+F71+F275,142+E82+F275, S43+K62+L,65, S43+K62+F71, S43+K62+E82, S43+K62+F275, S43+L65+F71, S43+L65+E82, S43+L65+F275, S43+F71+E82, S43+F71+F275, S43+E82+F275, K62+L65+F71, K62+L65+E82, K62+L65+F275, K62+F71+E82, K62+F71 F275, K62+E82+F275, L65+F71+E82, L65+F71+F275, L65+E82+F275 and F71+E82+F275, preferably substitutions in positions selected from the group consisting of K62+L65+F71, S43+L65+F71, S43+K62+L65 and S43+K62+F71, in particular S43+K62+L65.

More particularly, the variant of the invention may comprise at least three (such as three) substitutions selected from the group consisting of F71Y, K62E and S43Q. Specific examples include L65Q+F71Y+K62E, L65Q+F71Y+S43Q, L65Q+K62E+S43Q and F71Y+K62E+S43Q, in particular L65Q+K62E+S43Q.

The variants of the invention possess an increased clotting activity (or a reduced clotting time) as compared to hFVIIa or rhFVIIa. In a preferred embodiment of the invention the ratio between the time to reach clot formation for the variant (tᵥₐᵣᵢₐₙₜ) and the time to reach clot formation for hFVIIa or rhFVIIa (t_{wt}) is at the most 0.9 when assayed in the "Whole Blood Assay" described herein. More preferably the ratio (t_{variant/twt}) is at the most 0.75, such as 0.7, even more preferably the ratio (t_{variant/twt}) is at the most 0.6, most preferably the ratio (t_{variant/twt}) is at the most 0.5 when assayed in the "Whole Blood Assay" described herein.

In a further interesting embodiment of the invention, the variant comprises 1-10 amino acid modifications (e.g. substitutions), such as 1-5 amino acid modifications (e.g. substitutions), e.g. 1-3 amino acid modifications (e.g. substitutions) relative to SEQ ID NO:1.

For example, the variant may contain at least one amino acid modification made in the Gla domain as explained in the section entitled "Modifications in the Gla domain" below, and/or at least one amino acid modification which leads to introduction of an N-glycosylation site as explained in the section entitled "Introduction of additional sugar moieties" below, and/or at least one amino acid modification which decreases the TFPI-binding affinity. Examples of the latter modifications are described in the section entitled "Other modifications" below.

### Further modifications

As indicated above the FVII or FVIIa variant of the invention may comprise further modifications, in particular further modifications which confer additional advantageous properties to the FVII or FVIIa molecule. Thus, in addition to one or more of the substitutions mentioned above, i.e. a substitution in one or more of the positions L39,142, S43, K62, L65, F71, E82, F275H, the variant may comprise at least one further amino acid modification, in particular at least one further amino acid substitution.

In order to avoid too much disruption of the structure and function of the FVII or FVIIa polypeptide, the FVII or FVIIa polypeptide variant of the invention typically comprises an amino acid sequence having at least 95% identity with SEQ ID NO:1, such as at least 96% identity with SEQ ID NO:1, e.g. at least 97% identity with SEQ ID NO: 1, at least 98% identity with SEQ ID NO:1, or at least 99% identity with SEQ ID NO:1. Amino acid sequence identity is conveniently determined from aligned sequences, using e.g. the ClustalW program, version 1.8, June 1999, using default parameters (Thompson et al., 1994, ClustalW: Improving the sensitivity of progressive multiple sequence alignment through sequence weighting, position-specific gap penalties and weight matrix choice, Nucleic Acids Research, 22: 4673-4680) or from the PFAM families database version 4.0 (http:Hl2fam.wustl.edu/) (Nucleic Acids Res. 1999 Jan 1; 27(1):260-2) by use of GENEDOC version 2.5 (Nicholas, K.B., Nicholas H.B. Jr., and Deerfield, D.W. II. 1997 GeneDoc: Analysis and Visualization of Genetic Variation, EMBNEW.NEWS 4:14; Nicholas, K.B. and Nicholas H.B. Jr. 1997 GeneDoc: Analysis and Visualization of Genetic Variation).

### Modifications in the Gla domain

In an interesting embodiment of the invention, the variant further comprises at least one amino acid modification (such as at least one amino acid substitution and/or insertion) in the Gla domain. Preferably, no modifications are made in position 6, 7, 14, 16, 19, 20, 25, 26, 29 and 35.

Without being limited by any particular theory, it is presently believed that an increased clotting activity may be achieved by an enhanced binding affinity of the FVIIa molecule to the phospholipid membranes present on the surface of activated platelets. This enhanced affinity is believed to result in a higher local concentration of the activated FVIIa polypeptide in close proximity to the other coagulation factors, particularly FX. Thus, the rate of activation of FX to FXa will be higher, simply due to a higher molar ratio of the activated FVII polypeptide to FX. The increased activation rate of FX then results in a higher amount of active thrombin, and thus a higher rate of cross-linking of fibrin.

Thus, in a preferred embodiment according to this aspect of the invention, the polypeptide variant has, in its activated form, an enhanced phospholipid membrane binding affinity relative to the rhFVIIa polypeptide. Phospholipid membrane binding affinity may be measured by methods known in the art, such as by the assays described in Nelsestuen et al., Biochemistry 1977; 30;10819-10824 or as described in Example 1 in US 6,017,882.

Modifications in the FVII Gla domain leading to an increased phospholipid membrane binding affinity have been described in the art (see, for example, WO 99/20767 and WO 00/66753). Particular interesting positions in the Gla domain to be modified are positions P10, K32, D33, A34 as well as insertion of an amino acid residue between A3 and F4.

Thus, in a preferred embodiment of the invention, the variant comprises, in addition to one or more of the modifications mentioned above, a substititution in a position selected from the group consisting of P10, K32, D33 and A34 and combinations thereof.

In another interesting embodiment at least one of said substitutions are combined with an insertion of an amino acid residue between position A3 and F4.

Particularly preferred positions are P10 and K32, i.e. in a particular interesting embodiment of the invention substitutions are made in positions P10 and K32, preferably P10Q+K32E.

Preferably, the substitution to be made in position 32 is K32E, the substitution to be made in position 10 is P10Q, the substitution to be made in position 33 is D33F, and the substitution to be made in position 34 is A34E. The amino acid residue to be inserted between position A3 and F4 is preferably a hydrophobic amino acid residue, in particular the insertion is A3AY. In an interesting embodiment of the invention the variant comprises at least one of the following further modifications: A3AY, P10Q, K32E, D33F, A34E or combinations thereof. Most preferably, the variant comprises one of the following further modifications: K32E, P10Q+K32E, A3AY+P10Q+K32E, A3AY+P10Q+K32E+D33F, A3AY+P10Q+K32E+A34E or A3AY+P10Q+K32E+D33F+A34E.

### Modifications outside the Gla domain

A circulating rhFVIIa half-life of 2.3 hours was reported in "Summary Basis for Approval for NovoSeven®", FDA reference number 96-0597. Relatively high doses and frequent administration are necessary to reach and sustain the desired therapeutic or prophylactic effect. As a consequence adequate dose regulation is difficult to obtain and the need of frequent intravenous administrations imposes restrictions on the patient's way of living.

A molecule with a longer circulation half-life and/or increased bioavailability (such as an increased Area Under the Curve as compared to rhFVIIa when administered intravenously) would decrease the number of necessary administrations. Given the association of the current rhFVIIa product with frequent injections, and the potential for obtaining more optimal therapeutic FVIIa levels with concomitant enhanced therapeutic effect, there is a clear need for improved FVII- or FVIIa-like molecules.

Accordingly, a further object of the present invention is to provide improved FVII or FVII molecules (FVII or FVIIa variants) with an increased half-life and/or an increased bioavailability (such as an increased Area Under the Curve as compared to rhFVIIa, when administered intravenously) and which has an increased clotting activity.

Accordingly, in an interesting embodiment of the invention, the variant of the invention further comprises at least one introduced attachment group for a non-polypeptide moiety, where said attachment group has been introduced in a position located outside the Gla domain.

Thus, an interesting variant of the invention is a variant which, in its activated form and when compared to rhFVIIa, generates in increased Area Under the Curve when administered intravenously (AUCᵢᵥ,), in particular when administered intravenously in rats. More particularly, interesting variants of the present invention are such variants where the ratio between the AUCᵢᵥ of said variant, in its actvated form, and the AUCᵢᵥ of rhFVIIa is at least 1.25, such as at least 1.5, e.g. at least 1.75, more preferably at least 2, such as at least 3, even more preferably at least 4, such as at least 5, in particular when administered (intravenously) in rats.

This effect may correspond to an increased functional *in vivo* half-life and/or an increased serum half-life as compared to rhFVIIa. Accordingly, in another interesting embodiment of the invention, the ratio between the functional *in vivo* half-life or the serum half-life for the variant, in its activated form, and the functional *in vivo* half-life or the serum half-life for rhFVIIa is at least 1.25. More preferably, the ratio between the relevant half-life for the variant, in its activated form, and the relevant half-life for rhFVIIa is at least 1.5, such as at least 1.75, e.g. at least 2, even more preferably at least 3, such as at least 4, e.g. at least 5.

One way to increase the circulation half-life of a protein is to ensure that renal clearance of the protein is reduced. This may be achieved by conjugating the protein to a chemical moiety, which is capable of conferring reduced renal clearance to the protein.

Furthermore, attachment of a chemical moiety to the protein or substitution of amino acids exposed to proteolysis may effectively block a proteolytic enzyme from contact leading to proteolytic degradation of the protein. Polyethylene glycol (PEG) is one such chemical moiety that has been used in the preparation of therapeutic protein products. WO 98/32466 suggests that FVII, among many other proteins, may be PEGylated but does not contain any further information in this respect. WO 01/58935 discloses a new strategy for developing FVII or FVIIa molecules having *inter alia* an increased half-life.

A number of suitable modifications leading to an increase in AUCᵢᵥ, functional *in vivo* half-life and/or serum half-life are disclosed in WO 01/58935. The variants disclosed in WO 01/58935 are the result of a generally new strategy for developing improved FVII or FVIIa molecules. The specific modifications described in WO 01/58935 may advantageously be combined with the modifications described previously herein.

The polypeptide variant may also be attached to a serine proteinase inhibitor to inhibit the catalytic site of the polypeptide variant. Alternatively, one or more of the amino acid residues present in the catalytic site (S344, D242 and H193) may be mutated in order to render the resulting variant inactive. One example of such a mutation includes S344A.

The introduced amino acid residue comprising an attachment group for a non-polypeptide moiety is selected on the basis of the nature of the non-polypeptide moiety of choice and, in most instances, on the basis of the method in which conjugation between the polypeptide variant and the non-polypeptide moiety is to be achieved. For instance, when the non-polypeptide moiety is a polymer molecule such as a polyethylene glycol or polyalkylene oxide derived molecule, amino acid residues comprising an attachment group may be selected from the group consisting of lysine, cysteine, aspartic acid, glutamic acid, histidine, and tyrosine, preferably lysine, cysteine, aspartic acid and glutamic acid, more preferably lysine and cysteine, in particular cysteine.

Whenever an attachment group for a non-polypeptide moiety is to be introduced into the parent polypeptide, the position of the amino acid residue to be modified is preferably located at the surface of the parent FVII or FVIIa polypeptide, and more preferably occupied by an amino acid residue which has at least 25% of its side chain exposed to the surface (as defined in Example 1 herein), preferably at least 50% of its side chain exposed to the surface (as defined in Example 1 herein). Such positions have been identified on the basis of an analysis of a 3D structure of the hFVII or hFVIIa molecule as described in the Materials and Methods section herein.

Furthermore, the position to be modified according to this aspect of the invention is preferably selected from a part of the FVII or FVIIa molecule that is located outside the tissue factor binding site, and/or outside the active site region, and/or outside the ridge of the active site binding cleft. These sites/regions are identified in Example 1 herein. It should be emphasized, however, that in certain situations, e.g. in case an inactivated polypeptide variant is desired, it may be advantageous to perform modifications in or close to such regions. For example, it is contemplated that one or more attachment groups for the non-polypeptide moieties, such as attachment groups for N-glycosylation sites, may advantageously be introduced in the active site region or at the ridge of the active site binding cleft of the FVII or FVIIa molecule. The active site region, the tissue factor binding site and the ridge of the active site binding cleft are defined in Example 1 herein and are constituted by the following residues:

I153, Q167, V168, L169, L170, L171, Q176, L177, C178, G179, G180, T181, V188, V189, S190, A191, A192, H193, C194, F195, D196, K197, I198, W201, V228, I229, I230, P231, S232, T233, Y234, V235, P236, G237, T238, T239, N240, H241, D242, I243, A244, L245, L246, V281, S282, G283, W284, G285, Q286, T293, T324, E325, Y326, M327, F328, D338, S339, C340, K341, G342, D343, S344, G345, G346, P347, H348, L358, T359, G360, I361, V362, S363, W364, G365, C368, V376, Y377, T378, R379, V380, Q382, Y383, W386, L387, L400 and F405 (active site region);

L13, K18, F31, E35, R36, L39, F40, I42, S43, S60, K62, D63, Q64, L65, I69, C70, F71, C72, L73, P74, F76, E77, G78, R79, E82, K85, Q88, I90, V92, N93, E94, R271, A274, F275, V276, R277, F278, R304, L305, M306, T307, Q308, D309, Q312, Q313, E325 and R379 (tissue factor binding site); and

N173, A175, K199, N200, N203, D289, R290, G291, A292, P321 and T370 (the ridge of the active site binding cleft).

In order to determine an optimal distribution of attachment groups, the distance between amino acid residues located at the surface of the FVII or FVIIa polypeptide is calculated on the basis of a 3D structure of the hFVII or hFVIIa polypeptide. More specifically, the distance between the CB's of the amino acid residues comprising such attachment groups, or the distance between the functional group (NZ for lysine, CG for aspartic acid, CD for glutamic acid, SG for cysteine) of one and the CB of another amino acid residue comprising an attachment group are determined. In case of glycine, CA is used instead of CB. In the FVII or FVIIa part of the polypeptide variant of the invention, any of said distances is preferably more than 8 Å, in particular more than 10Å in order to avoid or reduce heterogeneous conjugation.

In case of introduction of an attachment group, an amino acid residue comprising such group is introduced into the position, preferably by substitution of the amino acid residue occupying such position.

The exact number of attachment groups present and available for conjugation in the FVII or FVIIa polypeptide is dependent on the effect desired to be achieved by the conjugation. The effect to be obtained is, e.g., dependent on the nature and degree of conjugation (e.g. the identity of the non-polypeptide moiety, the number of non-polypeptide moieties desirable or possible to conjugate to the polypeptide variant, where they should be conjugated or where conjugation should be avoided, etc.).

Functional *in vivo* half-life is *inter alia* dependent on the molecular weight of the protein, and the number of attachment groups needed for providing increased half-life thus depends on the molecular weight of the non-polypeptide moiety in question. In one embodiment, the polypeptide variant of the invention has a molecular weight of at least 67 kDa, in particular at least 70 kDa, e.g., as measured by SDS-PAGE according to Laemmli, U.K., Nature Vol 227 (1970), p680-85. FVII itself has a molecular weight of about 53 kDa, and therefore additional 10-20kDa is required to obtain the desired effect. This may, e.g., be provided by conjugating 2-4 10kDa PEG molecules or as otherwise described herein.

The total number of amino acid residues to be modified outside the Gla domain in the parent FVII or FVIIa polypeptide (as compared to the amino acid sequence shown in SEQ ID NO:1) will typically not exceed 10. Preferably, the FVII or FVIIa variant comprises an amino acid sequence which differs in 1-10 amino acid residues from amino acid residues 46-406 shown in SEQ ID NO:1, typically in 1-8 or in 2-8 amino acid residues, e.g. in 1-5 or in 2-5 amino acid residues, such as in 1-4 or in 1-3 amino acid residues, e.g. in 1, 2 or 3 amino acid residues from amino acid residues 46-406 shown in SEQ ID NO:1.

Analogously, the polypeptide variant of the invention may contain 1-10 (additional) non-polypeptide moieties, typically 1-8 or 2-8 (additional) non-polypeptide moieties, preferably 1-5 or 2-5 (additional) non-polypeptide moieties, such as 1-4 or 1-3 (additional) non-polypeptide moieties, e.g. 1, 2 or 3 (additional) non-polypeptide moieties. It will be understood that such additional non-polypeptide moieties are covalently attached to an attachment group located outside the Gla domain.

### Introduction of additional sugar moieties

In a preferred embodiment of the invention, an attachment group for a sugar moiety, such as a glycosylation site, in particular an *in vivo* glycosylation site, such as an N-glycosylation site, has been introduced in a position located outside the Gla domain.

When used in the present context, the term "naturally occurring glycosylation site" covers the glycosylation sites at postions N145, N322, S52 and S60. In a similar way, the term "naturally occurring O-glycosylation site" includes the positions S52 and S60, whereas the term "naturally occurring N-glycosylation site" includes positions N145 and N322.

Thus, in a very interesting embodiment of the invention, the non-polypeptide moiety is a sugar moiety and the introduced attachment group is a glycosylation site, preferably an *in vivo* glycosylation site, such as an O-glycosylation site or an N-glycosylation site, in particular an N-glycosylation site. Typically, 1-10 glycosylation sites, in particular N-glycosylation sites, have been introduced, preferably 1-8, 1-6, 1-4 or 1-3 glycosylation sites, in particular N-glycosylation sites, have been introduced in (a) positions(s) located outside the Gla domain. For example 1, 2 or 3 glycosylation sites, in particular N-glycosylation sites, may have been introduced outside the Gla domain, preferably by substitution. Analogously, the variant may comprise 1-10 introduced sugar moieties, preferably 1-8, 1-6, 1-4 or 1-3 introduced sugar moieties. For example, the variant may contain 1, 2 or 3 introduced sugar moieties.

It will be understood that in order to prepare a polypeptide variant, wherein the polypeptide variant comprises one or more glycosylation sites, the polypeptide variant must be expressed in a host cell capable of attaching sugar (oligosaccharide) moieties at the glycosylation site(s) or alternatively subjected to *in vitro* glycosylation. Examples of glycosylating host cells are given in the section further below entitled "Coupling to a sugar moiety".

Examples of positions, wherein the glycosylation sites, in particular N-glycosylation sites, may be introduced include, but is not limited to, positions comprising an amino acid residue having an amino acid residue having at least 25% of its side chain exposed to the surface (as defined in Example 1 herein), such as in a position comprising an amino acid residue having at least 50% of its side chain exposed to the surface (as defined in Example 1 herein). The position is preferably selected from a part of the molecule that is located outside the tissue factor binding site and/or the active site region and/or outside the ridge of the active site cleft. These sites/regions are identified in Example 1 herein. It should be understood that when the term "at least 25% (or at least 50%) of its side chain exposed to the surface" is used in connection with introduction of an N-glycosylation site this term refers to the surface accessibility of the amino acid side chain in the position where the sugar moiety is actually attached. In many cases it will be necessary to introduce a serine or a threonine residue in position +2 relative to the asparagine residue to which the sugar moiety is actually attached (unless, of course, this position is already occupied by a serine or a threonine residue) and these positions, where the serine or threonine residues are introduced, are allowed to be buried, i.e. to have less than 25% of their side chains exposed to the surface.

Specific and preferred examples of such substitutions creating an N-glycosylation site include a substitution selected from the group consisting of A51N, G58N, G58N+S60T, T106N, K109N, G124N, K143N+N145T, A175T, I205S, I205T, V253N, T267N, T267N+S269T, S314N+K316S, S314N+K316T, R315N+V317S, R315N+V317T, K316N+G318S, K316N+G318T, G318N, D334N and combinations thereof. More preferably, the N-glycosylation site is introduced by a substitution selected from the group consisting of A51N, G58N+S60T, T106N, K109N, G124N, K143N+N145T, A175T, I205T, V253N, T267N+S269T, S314N+K316T, R315N+V317T, K316N+G318T, G318N, D334N and combinations thereof. Even more preferably, the N-glycosylation site is introduced by a substitution selected from the group consisting of T106N, A175T, I205T, V253N, T267N+S269T and combinations thereof. Most preferably, the N-glycosylation site is introduced by a substitution selected from the group consisting of T106N, I205T, V253N, T267N+S269T and combinations thereof.

In one embodiment, only one N-glycosylation site has been introduced by substitution. In another embodiment, two or more (such as two) N-glycosylation sites have been introduced by substitution. Examples of preferred substitutions creating two N-glycosylation sites include substitutions selected from the group consisting of A51N+G58N, AS1N+G58N+S60T, A51N+T106N, AS1N+K109N, A51N+G124N, A51N+K143N+N145T, A51N+A175T, AS1N+I205T, A51N+V253N, A51N+T267N+S269T, AS1N+S314N+K316T, A51N+R315N+V317T, A51N+K316N+G318T, A51N+G318N, AS1N+D334N, G58N+T106N, G58N+K109N, G58N+G124N, G58N+K143N+N145T, G58N+A175T, G58N+I205T, G58N+V253N, G58N+T267N+S269T, G58N+S314N+K316T, G58N+R315N+V317T, G58N+K316N+G318T, G58N+G318N, G58N+D334N, G58N+S60T+T106N, G58N+S60T+K109N, G58N+S60T+G124N, G58N+S60T+K143N+ N145T, G58N+S60T+A175T, G58N+S60T+I205T, G58N+S60T+V253N, G58N+S60T+ T267N+S269T, G58N+S60T+S314N+K316T, G58N+S60T+R315N+V317T, G58N+S60T+K316N+G318T, G58N+S60T+G318N, G58N+S60T+D334N, T106N+K109N, T106N+G124N, T106N+K143N+N145T, T106N+A175T, T106N+I205T, T106N+V253N, T106N+T267N+S269T, T106N+S314N+K316T, T106N+R315N+V317T, T106N+K316N+G318T, T106N+G318N, T106N+D334N, K109N+G124N, K109N+K143N+N145T, K109N+A175T, K109N+I205T, K109N+V253N, K109N+T267N+S269T, K109N+S314N+K316T, K109N+R315N+V317T, K109N+K316N+G318T, K109N+G318N, K109N+D334N, G124N+K143N+N145T, G124N+A175T, G124N+I205T, G124N+V253N, G124N+T267N+S269T, G124N+S314N+K316T, G124N+R315N+V317T, G124N+K316N+G318T, G124N+G318N, G124N+D334N, K143N+N145T+A175T, K143N+N145T+I205T, K143N+N145T+V253N, K143N+N145T+T267N+S269T, K143N+N145T+S314N+K316T, K143N+N145T+R315N+V317T, K143N+N145T+K316N+G318T, K143N+N145T+G318N, K143N+N145T+D334N, A175T+I205T, A175T+V253N, A175T+T267N+S269T, A175T+S314N+K316T, A17ST+R315N+V317T, A175T+K316N+G318T, A175T+G318N, A175T+D334N, I205T+V253N, I205T+T267N+S269T, I205T+S314N+K316T, I205T+R315N+V317T, I205T+K316N+G318T, I205T+G318N, I205T+D334N, V253N+T267N+S269T, V253N+S314N+K316T, V253N+R315N+V317T, V253N+K316N+G318T, V253N+G318N, V253N+D334N, T267N+S269T+S314N+K316T, T267N+S269T+R315N+V317T, T267N+S269T+K316N+G318T, T267N+S269T+G318N, T267N+S269T+D334N, S314N+K316T+R315N+V317T, S314N+K316T+G318N, S314N+K316T+D334N, R315N+V317T+K316N+G318T, R315N+V317T+G318N, R315N+V317T+D334N and G318N+D334N. More preferably, the substitutions are selected from the group consisiting of T106N+A175T, T106N+I205T, T106N+V253N, T106N+T267N+S269T, A175T+I205T, A175T+V253N, A175T+T267N+S269T, I205T+V253N, I205T+T267N+S269T and V253N+T267N+S269T, even more preferably from the group consisiting of T106N+I205T, T106N+V253N, T106N+T267N+S269T, I205T+V253N, I205T+T267N+S269T and V253N+T267N+S269T.

In an even further embodiment, three or more (such as three) N-glycosylation sites have been introduced by substitution. Examples of preferred substitutions creating three N-glycosylation sites include substitutions selected from the group consisiting of T106N+A175T+I205T, T106N+A175T+V253N, T106N+A175T+T267N+S269T, T106N+I205T+V253N, T106N+I205T+T267N+S269T, T106N+V253N+T267N+S269T, A175T+I205T+V253N, A175T+I205T+T267N+S269T, A175T+V253N+T267N+S269T and I205T+V253N+T267N+S269T. More preferably, the substitutions are selected from the group consisting of T106N+1205T+V253N, T106N+1205T+T267N+S269T, T106N+V253N+T267N+S269T and I205T+V253N+T267N+S269T.

As discussed above, it is preferred that the N-glycosylation site is introduced in a position which does neither form part of the tissue factor binding site nor form part of the active site region and the ridge of the active site binding cleft as defined herein. It is envisaged that such glycosylation variants will primarily belong to the class of active polypeptide variants as defined hereinbefore.

It will be understood that any of the modifications mentioned in the above sections may be combined.

### Other modifications

In a further embodiment of the present invention, the FVII or FVIIa variant may, in addition to the modifications described in the sections above, also contain mutations, which are known to increase the intrinsic activity of the polypeptide, e.g. such as those described in WO 02/22776

Examples of preferred substitutions include substitutions selected from the group consisting of V158D, E296D, M298Q, L305V and K337A. More preferably, said substitutions are selected from the group consisting of V158D+E296D+M298Q+L305V+K337A, V158D+E296D+M298Q+K337A, V158D+E296D+M298Q+L305V, V 158D+E296D+M298Q, M298Q, L305V+K337A, L305V and K337A.

In a further embodiment of the present invention, the FVII or FVIIa variant may, in addition to the modifications described in the sections above, also contain mutations, which cause a decreased inhibition by TFPI. One example includes the substitution K341Q disclosed by Neuenschwander et al., Biochemistry 1995; 34:8701-8707. Other examples include D196K, D196N, G237L, G237GAA and combinations thereof.

As already indicated above, the variant may also contain conservative amino acid substitutions.

### Specific examples of most the preferred variants of the invention

Specifc examples of the most preferred variants of FVII or FVIIa are given below: S43Q, K62E, L65Q, S43Q+K62E, S43Q+L65Q, K62E+L65Q, S43Q+K62E+L65Q, P10Q+K32E+S43Q, P10Q+K32E+K62E, P10Q+K32E+L65Q, P10Q+K32E+S43Q+K62E, P10Q+K32E+S43Q+L65Q, P10Q+K32E+K62E+L65Q, P10Q+K32E+S43Q+K62E+L65Q

It will be understood that any of the above-mentioned preferred variants may be combined with at least one further modification performed outside the Gla domain. In particular any of the above-mentioned preferred variants may be combined with (a) substitution(s) selected from the group consisting of T106N, I205T, V253N, T267N+S269T, T106N+I205T, T106N+V253N, T106N+T267N+S269T, I205T+V253N, I205T+T267N+S269T, V253N+T267N+S269T, T106N+I205T+V253N, T106N+I205T+T267N+S269T, T106N+V253N+T267N+S269T and I205T+V253N+T267N+S269T.

### The non-polypeptide moiety

Based on the present disclosure the skilled person will be aware that amino acid residues comprising other attachment groups may be introduced by substitution into the parent polypeptide, using the same approach as that illustrated above with N-glycosylation sites. For instance, one or more amino acid residues comprising an acid group (glutamic acid or aspartic acid), tyrosine or lysine may be introduced into the positions discussed above. In particular, one or more cysteine residues may be introduced in the positions discussed above.

As indicated further above the non-polypeptide moiety of the conjugated variant is preferably selected from the group consisting of a polymer molecule, a lipophilic compound, a sugar moiety (by way of *in vivo* glycosylation) and an organic derivatizing agent. All of these agents may confer desirable properties to the variant polypeptide, in particular increased AUCᵢᵥ, increased functional *in vivo* half-life and/or increased plasma half-life. The variant polypeptide is normally conjugated to only one type of non-polypeptide moiety, but may also be conjugated to two or more different types of non-polypeptide moieties, e.g. to a polymer molecule and a sugar moiety, to a lipophilic group and a sugar moiety, to an organic derivatizing agent and a sugar moiety, to a lipophilic group and a polymer molecule, etc. The conjugation to two or more different non-polypeptide moieties may be done simultaneous or sequentially.

### Methods of preparing a conjugated variant of the invention.

In the following sections *"Conjugation to a polymer molecule", "Conjugation to a sugar moiety ", "Conjugation to an organic derivatizing agent* " and *"Conjugation to a lipophilic compound",* conjugation to specific types of non-polypeptide moieties is described. In general, a conjugated variant according to the invention may be produced by culturing an appropriate host cell under conditions conducive for the expression of the variant polypeptide, and recovering the variant polypeptide, wherein a) the variant polypeptide comprises at least one N- or O-glycosylation site and the host cell is an eukaryotic host cell capable of *in vivo* glycosylation, and/or b) the variant polypeptide is subjected to conjugation to a non-polypeptide moiety *in vitro.*

It will be understood that the conjugation should be designed so as to produce the optimal molecule with respect to the number of non-polypeptide moieties attached, the size and form of such molecules (e.g. whether they are linear or branched), and the attachment site(s) in the polypeptide. The molecular weight of the non-polypeptide moiety to be used may, e.g., be chosen on the basis of the desired effect to be achieved. For instance, if the primary purpose of the conjugation is to achieve a conjugated variant having a high molecular weight (e.g. to reduce renal clearance) it is usually desirable to conjugate as few high molecular weight non-polypeptide moieties as possible to obtain the desired molecular weight. When a high degree of shielding is desirable this may be obtained by use of a sufficiently high number of low molecular weight non-polypeptide moieties (e.g. with a molecular weight of from about 300 Da to about 5 kDa, such as a molecular weight of from 300 Da to 2 kDa).

### Conjugation to a polymer molecule

The polymer molecule to be coupled to the variant polypeptide may be any suitable polymer molecule, such as a natural or synthetic homo-polymer or hetero-polymer, typically with a molecular weight in the range of about 300-100,000 Da, such as about 500-20,000 Da, more preferably in the range of about 500-15,000 Da, even more preferably in the range of about 2-12 kDa, such as in the range of about 3-10 kDa. When the term "about" is used herein in connection with a certain molecular weight, the word "about" indicates an approximate average molecular weight and reflects the fact that there will normally be a certain molecular weight distribution in a given polymer preparation.

Examples of homo-polymers include a polyol (i.e. poly-OH), a polyamine (i.e. poly-NH₂) and a polycarboxylic acid (i.e. poly-COOH). A hetero-polymer is a polymer comprising different coupling groups, such as a hydroxyl group and an amine group.

Examples of suitable polymer molecules include polymer molecules selected from the group consisting of polyalkylene oxide (PAO), including polyalkylene glycol (PAG), such as polyethylene glycol (PEG) and polypropylene glycol (PPG), branched PEGs, poly-vinyl alcohol (PVA), poly-carboxylate, poly-(vinylpyrolidone), polyethylene-co-maleic acid anhydride, polystyrene-co-maleic acid anhydride, dextran, including carboxymethyl-dextran, or any other biopolymer suitable for reducing immunogenicity and/or increasing functional *in vivo* half-life and/or serum half-life. Another example of a polymer molecule is human albumin or another abundant plasma protein. Generally, polyalkylene glycol-derived polymers are biocompatible, non-toxic, non-antigenic, non-immunogenic, have various water solubility properties, and are easily excreted from living organisms.

PEG is the preferred polymer molecule, since it has only few reactive groups capable of cross-linking compared to, e.g., polysaccharides such as dextran. In particular, monofunctional PEG, e.g. methoxypolyethylene glycol (mPEG), is of interest since its coupling chemistry is relatively simple (only one reactive group is available for conjugating with attachment groups on the polypeptide). Consequently, as the risk of cross-linking is eliminated, the resulting conjugated variants are more homogeneous and the reaction of the polymer molecules with the variant polypeptide is easier to control.

To effect covalent attachment of the polymer molecule(s) to the variant polypeptide, the hydroxyl end groups of the polymer molecule must be provided in activated form, i.e. with reactive functional groups (examples of which include primary amino groups, hydrazide (HZ), thiol, succinate (SUC), succinimidyl succinate (SS), succinimidyl succinamide (SSA), succinimidyl propionate (SPA), succinimidyl butyrate (SBA), succinimidy carboxymethylate (SCM), benzotriazole carbonate (BTC), N-hydroxysuccinimide (NHS), aldehyde, nitrophenylcarbonate (NPC), and tresylate (TRES)). Suitable activated polymer molecules are commercially available, e.g. from Shearwater Polymers, Inc., Huntsville, AL, USA, or from PolyMASC Pharmaceuticals plc, UK.

Alternatively, the polymer molecules can be activated by conventional methods known in the art, e.g. as disclosed in WO 90/13540. Specific examples of activated linear or branched polymer molecules for use in the present invention are described in the Shearwater Polymers, Inc. 1997 and 2000 Catalogs (Functionalized Biocompatible Polymers for Research and pharmaceuticals, Polyethylene Glycol and Derivatives).

Specific examples of activated PEG polymers include the following linear PEGs: NHS-PEG (e.g. SPA-PEG, SSPA-PEG, SBA-PEG, SS-PEG, SSA-PEG, SC-PEG, SG-PEG, and SCM-PEG), and NOR-PEG, BTC-PEG, EPOX-PEG, NCO-PEG, NPC-PEG, CDI-PEG, ALD-PEG, TRES-PEG, VS-PEG, IODO-PEG, and MAL-PEG, and branched PEGs such as PEG2-NHS and those disclosed in US 5,932,462 and US 5,643,575.

Furthermore, the following publications disclose useful polymer molecules and/or PEGylation chemistries: US 5,824,778, US 5,476,653, WO 97/32607, EP 0 229 108, EP 0 402 378, US 4,902,502, US 5,281,698, US 5,122,614, US 5,219,564, WO 92/16555, WO 94/04193, WO 94/14758, WO 94/17039, WO 94/18247, WO 94/28024, WO 95/00162, WO 95/11924, WO95/13090, WO 95/33490, WO 96/00080, WO 97/18832, WO 98/41562, WO 98/48837, WO 99/32134, WO 99/32139, WO 99/32140, WO 96/40791, WO 98/32466, WO 95/06058, EP 0 439 508, WO 97/03106, WO 96/21469, WO 95/13312, EP 0 921 131, US 5,736,625, WO 98/05363, EP 0 809 996, US 5,629,384, WO 96/41813, WO 96/07670, US 5,473,034, US 5,516,673, EP 0 605 963, US 5,382,657, EP 0 510 356, EP 0 400 472, EP 0 183 503 and EP 0 154 316.

Specific examples of activated PEG polymers particularly preferred for coupling to cysteine residues, include the following linear PEGs: vinylsulfone-PEG (VS-PEG), preferably vinylsulfone-mPEG (VS-mPEG); maleimide-PEG (MAL-PEG), preferably maleimide-mPEG (MAL-mPEG) and orthopyridyl-disulfide-PEG (OPSS-PEG), preferably orthopyridyl-disulfide-mPEG (OPSS-mPEG). Typically, such PEG or mPEG polymers will have a size of about 5 kDa, about 10 kD, about 12 kDa or about 20 kDa.

The conjugation of the polypeptide variant and the activated polymer molecules is conducted by use of any conventional method, e.g. as described in the following references (which also describe suitable methods for activation of polymer molecules): Harris and Zalipsky, eds., Poly(ethylene glycol) Chemistry and Biological Applications, AZC, Washington; R.F. Taylor, (1991), "Protein immobilisation. Fundamental and applications", Marcel Dekker, N.Y.; S.S. Wong, (1992), "Chemistry of Protein Conjugation and Crosslinking", CRC Press, Boca Raton; G.T. Hermanson et al., (1993), "Immobilized Affinity Ligand Techniques", Academic Press, N.Y.).

The skilled person will be aware that the activation method and/or conjugation chemistry to be used depends on the attachment group(s) of the variant polypeptide (examples of which are given further above), as well as the functional groups of the polymer (e.g. being amine, hydroxyl, carboxyl, aldehyde, sulfydryl, succinimidyl, maleimide, vinysulfone or haloacetate). The PEGylation may be directed towards conjugation to all available attachment groups on the variant polypeptide (i.e. such attachment groups that are exposed at the surface of the polypeptide) or may be directed towards one or more specific attachment groups, e.g. the N-terminal amino group as described in US 5,985,265 or to cysteine residues. Furthermore, the conjugation may be achieved in one step or in a stepwise manner (e.g. as described in WO 99/55377).

For PEGylation to cysteine residues (see above) the FVII or FVIIa variant is usually treated with a reducing agent, such as dithiothreitol (DDT) prior to PEGylation. The reducing agent is subsequently removed by any conventional method, such as by desalting. Conjugation of PEG to a cysteine residue typically takes place in a suitable buffer at pH 6-9 at temperatures varying from 4°C to 25°C for periods up to 16 hours.

It will be understood that the PEGylation is designed so as to produce the optimal molecule with respect to the number of PEG molecules attached, the size and form of such molecules (e.g. whether they are linear or branched), and the attachment site(s) in the variant polypeptide. The molecular weight of the polymer to be used may e.g. be chosen on the basis of the desired effect to be achieved.

In connection with conjugation to only a single attachment group on the protein (e.g. the N-terminal amino group), it may be advantageous that the polymer molecule, which may be linear or branched, has a high molecular weight, preferably about 10-25 kDa, such as about 15-25 kDa, e.g. about 20 kDa.

Normally, the polymer conjugation is performed under conditions aimed at reacting as many of the available polymer attachment groups with polymer molecules. This is achieved by means of a suitable molar excess of the polymer relative to the polypeptide. Typically, the molar ratios of activated polymer molecules to polypeptide are up to about 1000-1, such as up to about 200-1, or up to about 100-1. In some cases the ration may be somewhat lower, however, such as up to about 50-1, 10-1, 5-1, 2-1 or 1-1 in order to obtain optimal reaction.

It is also contemplated according to the invention to couple the polymer molecules to the polypeptide through a linker. Suitable linkers are well known to the skilled person. A preferred example is cyanuric chloride (Abuchowski et al., J Biol Chem 1977;252;3578-3581; US 4,179,337; Shafer et al., J Polym Sci Polym Chem Ed 1986;24;375-378).

Subsequent to the conjugation, residual activated polymer molecules are blocked according to methods known in the art, e.g. by addition of primary amine to the reaction mixture, and the resulting inactivated polymer molecules are removed by a suitable method.

It will be understood that depending on the circumstances, e.g. the amino acid sequence of the variant polypeptide, the nature of the activated PEG compound being used and the specific PEGylation conditions, including the molar ratio of PEG to polypeptide, varying degrees of PEGylation may be obtained, with a higher degree of PEGylation generally being obtained with a higher ratio of PEG to variant polypeptide. The PEGylated variant polypeptides resulting from any given PEGylation process will, however, normally comprise a stochastic distribution of conjugated polypeptide variants having slightly different degrees of PEGylation.

### Conjugation to a sugar moiety

In order to achieve *in vivo* glycosylation of a FVII molecule comprising one or more glycosylation sites the nucleotide sequence encoding the variant polypeptide must be inserted in a glycosylating, eucaryotic expression host. The expression host cell may be selected from fungal (filamentous fungal or yeast), insect or animal cells or from transgenic plant cells. In one embodiment the host cell is a mammalian cell, such as a CHO cell, BHK or HEK, e.g. HEK 293, cell, or an insect cell, such as an SF9 cell, or a yeast cell, e.g. *S*. *cerevisiae* or *Pichia pastoris,* or any of the host cells mentioned hereinafter.

*Covalent in vitro* coupling of sugar moieties (such as dextran) to amino acid residues of the variant polypeptide may also be used, e.g. as described, for example in WO 87/05330 and in Aplin etl al., CRC Crit Rev. Biochem 1981;259-306. The *in vitro* coupling of sugar moieties or PEG to protein- and peptide-bound Gln-residues can be carried out by transglutaminases (TGases). Transglutaminases catalyse the transfer of donor amine-groups to protein- and peptide-bound Gln-residues in a so-called cross-linking reaction. The donor-amine groups can be protein- or peptide-bound , such as the ε-amino-group in Lys-residues or it can be part of a small or large organic molecule. An example of a small organic molecule functioning as amino-donor in TGase-catalysed cross-linking is putrescine (1,4-diaminobutane). An example of a larger organic molecule functioning as amino-donor in TGase-catalysed cross-linking is an amine-containing PEG (Sato et al., Biochemistry 1996;35;13072-13080).

TGases, in general, are highly specific enzymes, and not every Gln-residues exposed on the surface of a protein is accessible to TGase-catalysed cross-linking to amino-containing substances. On the contrary, only few Gln-residues are naturally functioning as TGase substrates but the exact parameters governing which Gln-residues are good TGase substrates remain unknown. Thus, in order to render a protein susceptible to TGase-catalysed cross-linking reactions it is often a prerequisite at convenient positions to add stretches of amino acid sequence known to function very well as TGase substrates. Several amino acid sequences are known to be or to contain excellent natural TGase substrates e.g. substance P, elafin, fibrinogen, fibronectin, α₂-plasmin inhibitor, α-caseins, and β-caseins.

### Conjugation to art organic derivatizing agent

Covalent modification of the variant polypeptide may be performed by reacting one or more attachment groups of the variant polypeptide with an organic derivatizing agent. Suitable derivatizing agents and methods are well known in the art. For example, cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(4-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole. Histidyl residues are derivatized by reaction with diethylpyrocarbonateat pH 5.5-7.0 because this agent is relatively specific for the histidyl side chain. Para-bromophenacyl bromide also is useful. The reaction is preferably performed in 0.1 M sodium cacodylate at pH 6.0. Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate, pyridoxal phosphate, pyridoxal, chloroborohydride, trinitrobenzenesulfonic acid, O-methylisourea, 2,4-pentanedione and transaminase-catalyzed reaction with glyoxylate. Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKa of the guanidine functional group.

Furthermore, these reagents may react with the groups of lysine as well as the arginine guanidino group. Carboxyl side groups (aspartyl or glutamyl) are selectively modified by reaction with carbodiimides (R-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

### Conjugation to a lipophilic compound

The variant polypeptide and the lipophilic compound may be conjugated to each other, either directly or by use of a linker. The lipophilic compound may be a natural compound such as a saturated or unsaturated fatty acid, a fatty acid diketone, a terpene, a prostaglandin, a vitamine, a carotenoide or steroide, or a synthetic compound such as a carbon acid, an alcohol, an amine and sulphonic acid with one or more alkyl-, aryl-, alkenyl- or other multiple unsaturated compounds. The conjugation between the variant polypeptide and the lipophilic compound, optionally through a linker may be done according to methods known in the art, e.g. as described by Bodanszky in Peptide Synthesis, John Wiley, New York, 1976 and in WO 96/12505.

### Attachment of serine protease inhibitor

Attachment of a serine protease inhibitor can be performed in accordance with the method described in WO 96/12800.

### Conjugation of a tagged polypeptide

In an alternative embodiment the polypeptide variant is expressed as a fusion protein with a tag, i.e. an amino acid sequence or peptide stretch made up of typically 1-30, such as 1-20 amino acid residues. Besides allowing for fast and easy purification, the tag is a convenient tool for achieving conjugation between the tagged polypeptide variant and the non-polypeptide moiety. In particular, the tag may be used for achieving conjugation in microtiter plates or other carriers, such as paramagnetic beads, to which the tagged polypeptide variant can be immobilised via the tag. The conjugation to the tagged polypeptide variant in, e.g., microtiter plates has the advantage that the tagged polypeptide variant can be immobilised in the microtiter plates directly from the culture broth (in principle without any purification) and subjected to conjugation. Thereby, the total number of process steps (from expression to conjugation) can be reduced. Furthermore, the tag may function as a spacer molecule, ensuring an improved accessibility to the immobilised polypeptide variant to be conjugated. The conjugation using a tagged polypeptide variant may be to any of the non-polypeptide moieties disclosed herein, e.g. to a polymer molecule such as PEG.

The identity of the specific tag to be used is not critical as long as the tag is capable of being expressed with the polypeptide variant and is capable of being immobilised on a suitable surface or carrier material. A number of suitable tags are commercially available, e.g. from Unizyme Laboratories, Denmark. The subsequent cleavage of the tag from the polypeptide variant may be achieved by use of commercially available enzymes.

### Inactivation of the FVII/FVIIa variants of the invention

In another interesting embodiment of the invention, the variants disclosed herein may be inactivated. The inactivated form is capable of competing with wild-type FVII or FVIIa for binding to TF and inhibiting clotting activity, and it is envisaged that such inactivated variants will be very potent tissue factor antagonists. Thus, in another aspect the present invention relates to the FVII/FVIIa variants described herein in their inactivated forms as well as to such inactivated FVII/FVIIa variants for use as medicaments. More particularly, the inactivated variant of the invention may be used for the manufacture of a medicament for the treatment or prophylaxis of a FVIIa/TF-related disease or disorder in a mammal. For example, the inactivated variant of the invention may be used for the manufacture of a medicament for the treatment or prophylaxis of diseases where anticoagulant activity is desirable, such as prophylaxis or treatment of patients being in hypercoagulable states, such as patients with sepsis, deep-vein thrombosis, patients in risk of myocardial infections or thrombotic stroke, pulmonary embolism, patients with acute coronary syndromes (myocardial infarction and unstable angina pectoris), patients undergoing coronary cardiac, prevention of cardiac events and restonosis for patients receiving angioplasty, patients with peripheral vascular diseases. The inactivated variant of the invention may also be used for the manufacture of a medicament for the treatment of respiratory diseases, tumor growth and metastasis. Analogously, the inactivated variant of the invention may be used in a method for treating a mammal having a FVIIa/TF-related disease or disorder (such as one or more of the diseases or disorders mentioned above), comprising administering to a mammal in need thereof an effective amount of such an inactivated conjugate or composition.

As used herein, the term "inactivated", when used in connection with the variants of the invention, is intended to mean a variant having less than 5% of the clotting activity of hFVIIa or rhFVIIa when measured in the "Whole Blood Assay" described herein.

The variants described herein may be inactivated by methods well-known in the art. For example, an active FVII or FVIIa polypeptide may be rendered inactive by carbamylating the α-amino acid group I153 or by complexing the polypeptide to a serine proteinase inhibitor, in accordance with the method described in WO 96/12800. A suitable serine inhibitor protein is, e.g., selected from the group consisting of an organophosphor compound, a sulfanylfluoride, a peptide halomethylketone, preferably a Dansyl-Phe-Pro-Arg chloromethylketone, Dansyl-Glu-Glu-Arg chlormethylketone, Dansyl-Phe-Phe-Arg chlormethylketone or a Phe-Phe-Arg chlormethylketone, or an azapeptide.

Alternatively, the variants of the invention may be rendered inactive by removing at least one amino acid residue occupying a position selected from the group consisting of R152, I153, S344, D242 and H193. The removal may be effected by substitution or deletion of one or more of the above-identified amino acid residues. Preferably, the removal is effected by substitution, in particular by conservative substitution. Accordingly, the inactivated FVII or FVIIa polypeptide used herein may comprise one or more of the following substitutions: R152X, I153X, S344X, D242X or H193X, wherein X is any amino acid residue, preferably one leading to a conservative substitution. For instance, the inactivated FVII or FVIIa polypeptide comprises the mutations R152X, wherein X is any amino acid residue other than lysine (since lysine forms part of a protease cleavage site). Other examples of specific substitutions include I153A/V/L; S344T/A/G/Y, preferably S344A; D242E/A and/or H193R/A.

Another approach includes performing modifications in or close to the active site region. For example, it is contemplated that one or more attachment groups for the non-polypeptide moieties, such as attachment groups for N-glycosylation sites, may advantageously be introduced in the active site region or at the ridge of the active site binding cleft of the FVII or FVIIa variant. The active site region, the tissue factor binding site and the ridge of the active site binding cleft are defined in Example I herein.

Thus, specific examples of substitutions creating such an N-glycosylation site include substitutions selected from the group consisting of I153N+G155S/T, Q167N+L169S/T, V168N+L170S/T, L169N+L171S/T, L170N+V172S/T, L171N+N173S/T, A175S/T, A175N+L177S/T, L177N+G179S/T, G179N, G180N+L182S/T, T181N+I183S/T, V188N, V189N+A191S/T, S190N+A192S/T, A191N+H193S/T, H193N+F195S/T, F195N+K197S/T, D196N+I198S/T, K197N+K199S/T, I198N+N200S/T, K199N+W201S/T, W201N+N203S/T, R202S/T, I205S/T, V228N+1230S/T, I229N+P231S/T, I230N, P231N, S232N+Y234S/T, T233N+V235S/T, Y234N+P236S/T, V235N+G237S/T, P236N, G237N, T238N+N240S/T, T239N+H241S/T, H241N+I243S/T, D242S/T, I243N+L245S/T, A244N+L246S/T, L245N+R247S/T, L246N+L246S/T, V281N+G283S/T, S282N+W284S/T, G283N+G285S/T, W284N+Q286S/T, G285N+L287S/T, Q286N+L288S/T, D289N+G291S/T, R290N+A292S/T, G291N, A292N+A294S/F, T293N+L295S/T, P321N+I323S/T, T324N+Y326S/T, Y326N+F327S/T, F328N+A330S/T, S339N+K341S/T, K341N+D343S/T, G342N+S344S/T, D343N+G345S/T, S344N+G346S/T, G345N+P347S/T, P347N+A349S/T, H348N, L358N+G360S/T, T359N+I361S/T, G360N+V362S/T, I361N, V362N+W364S/T, S363N+G365S/T, W364N+Q366S/T, G365N+G367S/T, T370N+G372S/T, V376N, Y377N+R379S/T, T378N+V380S/T, V380N+Q382S/T, Q382N+I384S/T, Y383N+E385S/T, W386N+Q388S/T, L387N+K389S/T, L400N+R402S/T and combinations thereof. Preferably, the substitution is selected from the group consisting of D289N+G291S/T, R290N+A292S/T, G291N, A292N+A294S/T, T293N+L295S/T, S339N+K341S/T, K341N+D343S/T, G342N+S344S/T, D343N+G345S/T, and combinations thereof. More preferably, the substitution is selected from the group consisting of D289N+G291T, R290N+A292T, G291N, A292N+A294T, T293N+L295T, S339N+K341T, K341N+D343T, G342N+S344T, D343N+G345T, and combinations thereof, in particular G291N.

Typically, an inactivated variant has significantly reduced clotting activity as compared to wild-type hFVIIa or rhFVIIa. Preferably, the inactivated variant has less than 4% of the clotting activity of hFVIIa or rhFVIIa when assayed in Whole Blood Assay described herein. More preferably the inactivated variant has less than 3% of the clotting activity, such as less than 2% of the clotting activity, e.g. less than 1% of the clotting activity of hFVIIa or rhFVIIa when assayed in the Whole Blood Assay described herein.

As will be understood, details and particulars concerning the inactivated variants of the invention (e.g. preferred substitutions, formulation of the variants, etc.) will be the same or analogous to the (active) variant aspect of the invention, whenever appropriate. Thus, statements and details concerning the inactivated variants of the invention will apply mutatis mutandis to the active variants disclosed herein, whenever appropriate.

### Methods of preparing a polypeptide variant of the invention

The polypeptide variants of the present invention, optionally in glycosylated form, may be produced by any suitable method known in the art. Such methods include constructing a nucleotide sequence encoding the polypeptide variant and expressing the sequence in a suitable transformed or transfected host. Preferably, the host cell is a gammacarboxylating host cell such as a mammalian cell. However, polypeptide variants of the invention may be produced, albeit less efficiently, by chemical synthesis or a combination of chemical synthesis or a combination of chemical synthesis and recombinant DNA technology.

A nucleotide sequence encoding a polypeptide variant of the invention may be constructed by isolating or synthesizing a nucleotide sequence encoding human wild-type FVII and then changing the nucleotide sequence so as to effect introduction (i.e. insertion or substitution) or removal (i.e. deletion or substitution) of the relevant amino acid residue(s).

The nucleotide sequence is conveniently modified by site-directed mutagenesis in accordance with conventional methods. Alternatively, the nucleotide sequence is prepared by chemical synthesis, e.g. by using an oligonucleotide synthesizer, wherein oligonucleotides are designed based on the amino acid sequence of the desired polypeptide variant, and preferably selecting those codons that are favored in the host cell in which the recombinant polypeptide variant will be produced. For example, several small oligonucleotides coding for portions of the desired polypeptide variant may be synthesized and assembled by PCR, ligation or ligation chain reaction (LCR) (Barany, PNAS 88:189-193, 1991). The individual oligonucleotides typically contain 5' or 3' overhangs for complementary assembly.

Alternative nucleotide sequence modification methods are available for producing polypeptide variants for high throughput screening, for instance methods which involve homologous cross-over such as disclosed in US 5,093,257, and methods which involve gene shuffling, i.e. recombination between two or more homologous nucleotide sequences resulting in new nucleotide sequences having a number of nucleotide alterations when compared to the starting nucleotide sequences. Gene shuffling (also known as DNA shuffling) involves one or more cycles of random fragmentation and reassembly of the nucleotide sequences, followed by screening to select nucleotide sequences encoding polypeptides with desired properties. In order for homology-based nucleic acid shuffling to take place, the relevant parts of the nucleotide sequences are preferably at least 50% identical, such as at least 60% identical, more preferably at least 70% identical, such as at least 80% identical. The recombination can be performed *in vitro* or *in vivo.*

Examples of suitable *in vitro* gene shuffling methods are disclosed by Stemmer et al. (1994), Proc. Natl. Acad. Sci. USA; vol. 91, pp. 10747-10751; Stemmer (1994), Nature, vol. 370, pp. 389-391; Smith (1994), Nature vol. 370, pp. 324-325; Zhao et al., Nat. Biotechnol. 1998, Mar; 16(3): 258-61; Zhao H. and Arnold, FB, Nucleic Acids Research, 1997, Vol. 25. No. 6 pp. 1307-1308; Shao et al., Nucleic Acids Research 1998, Jan 15; 26(2): pp. 681-83; and WO 95/17413.

An example of a suitable *in vivo* shuffling method is disclosed in WO 97/07205. Other techniques for mutagenesis of nucleic acid sequences by *in vitro* or *in vivo* recombination are disclosed e.g. in WO 97/20078 and US 5,837,458. Examples of specific shuffling techniques include "family shuffling", "synthetic shuffling" and *"in silico* shuffling".

Family shuffling involves subjecting a family of homologous genes from different species to one or more cycles of shuffling and subsequent screening or selection. Family shuffling techniques are disclosed e.g. by Crameri et al. (1998), Nature, vol. 391, pp. 288-291; Christians et al. (1999), Nature Biotechnology, vol. 17, pp. 259-264; Chang et al. (1999), Nature Biotechnology, vol. 17, pp. 793-797; and Ness et al. (1999), Nature Biotechnology, vol. 17, 893-896.

Synthetic shuffling involves providing libraries of overlapping synthetic oligonucleotides based e.g. on a sequence alignment of homologous genes of interest. The synthetically generated oligonucleotides are recombined, and the resulting recombinant nucleic acid sequences are screened and if desired used for further shuffling cycles. Synthetic shuffling techniques are disclosed in WO 00/42561.

*In silico* shuffling refers to a DNA shuffling procedure, which is performed or modelled using a computer system, thereby partly or entirely avoiding the need for physically manipulating nucleic acids. Techniques for *in silico* shuffling are disclosed in WO 00/42560.

Once assembled (by synthesis, site-directed mutagenesis or another method), the nucleotide sequence encoding the polypeptide is inserted into a recombinant vector and operably linked to control sequences necessary for expression of the FVII in the desired transformed host cell.

It should of course be understood that not all vectors and expression control sequences function equally well to express the nucleotide sequence encoding the polypeptide variants described herein. Neither will all hosts function equally well with the same expression system. However, one of skill in the art may make a selection among these vectors, expression control sequences and hosts without undue experimentation. For example, in selecting a vector, the host must be considered because the vector must replicate in it or be able to integrate into the chromosome. The vector's copy number, the ability to control that copy number, and the expression of any other proteins encoded by the vector, such as antibiotic markers, should also be considered. In selecting an expression control sequence, a variety of factors should also be considered. These include, for example, the relative strength of the sequence, its controllability, and its compatibility with the nucleotide sequence encoding the polypeptide, particularly as regards potential secondary structures. Hosts should be selected by consideration of their compatibility with the chosen vector, the toxicity of the product coded for by the nucleotide sequence, their secretion characteristics, their ability to fold the polypeptide variant correctly, their fermentation or culture requirements, and the ease of purification of the products coded for by the nucleotide sequence.

The recombinant vector may be an autonomously replicating vector, i.e. a vector, which exists as an extrachromosomal entity, the replication of which is independent of chromosomal replication, e.g. a plasmid. Alternatively, the vector is one which, when introduced into a host cell, is integrated into the host cell genome and replicated together with the chromosome(s) into which it has been integrated.

The vector is preferably an expression vector, in which the nucleotide sequence encoding the polypeptide variant of the invention is operably linked to additional segments required for transcription of the nucleotide sequence. The vector is typically derived from plasmid or viral DNA. A number of suitable expression vectors for expression in the host cells mentioned herein are commercially available or described in the literature. Useful expression vectors for eukaryotic hosts, include, for example, vectors comprising expression control sequences from SV40, bovine papilloma virus, adenovirus and cytomegalovirus. Specific vectors are, e.g., pCDNA3.1(+)\Hyg (Invitrogen, Carlsbad, CA, USA) and pCI-neo (Stratagene, La Jola, CA, USA). Useful expression vectors for yeast cells include the 2µ plasmid and derivatives thereof, the POT1 vector (US 4,931,373), the pJSO37 vector described in Okkels, Ann. New York Acad. Sci. 782, 202-207, 1996, and pPICZ A, B or C (Invitrogen). Useful vectors for insect cells include pVL941, pBG311 (Cate et al., "Isolation of the Bovine and Human Genes for Mullerian Inhibiting Substance And Expression of the Human Gene In Animal Cells", Cell, 45, pp. 685-98 (1986), pBluebac 4.5 and pMelbac (both available from Invitrogen). Useful expression vectors for bacterial hosts include known bacterial plasmids, such as plasmids from *E*. *coli,* including pBR322, pET3a and pET12a (both from Novagen Inc., WI, USA), wider host range plasmids, such as RP4, phage DNAs, e.g., the numerous derivatives of phage lambda, e.g., NM989, and other DNA phages, such as M13 and filamentous single stranded DNA phages.

Other vectors for use in this invention include those that allow the nucleotide sequence encoding the polypeptide variant to be amplified in copy number. Such amplifiable vectors are well known in the art. They include, for example, vectors able to be amplified by DHFR amplification (see, e.g., Kaufman, U.S. Pat. No. 4,470,461, Kaufman and Sharp, "Construction Of A Modular Dihydrafolate Reductase cDNA Gene: Analysis Of Signals Utilized For Efficient Expression", Mol. Cell. Biol., 2, pp. 1304-19 (1982)) and glutamine synthetase ("GS") amplification (see, e.g., US 5,122,464 and EP 338,841).

The recombinant vector may further comprise a DNA sequence enabling the vector to replicate in the host cell in question. An example of such a sequence (when the host cell is a mammalian cell) is the SV40 origin of replication. When the host cell is a yeast cell, suitable sequences enabling the vector to replicate are the yeast plasmid 2µ replication genes REP 1-3 and origin of replication.

The vector may also comprise a selectable marker, e.g. a gene the product of which complements a defect in the host cell, such as the gene coding for dihydrofolate reductase (DHFR) or the Schizosaccharomyces pombe TPI gene (described by P.R. Russell, Gene 40, 1985, pp. 125-130), or one which confers resistance to a drug, e.g. ampicillin, kanamycin, tetracyclin, chloramphenicol, neomycin, hygromycin or methotrexate. For *Saccharomyces cerevisiae,* selectable markers include *ura3 and leu2.* For filamentous fungi, selectable markers include *amdS, pyrG, arcS, niaD* and *sC.*

The term "control sequences" is defined herein to include all components, which are necessary or advantageous for the expression of the polypeptide variant of the invention. Each control sequence may be native or foreign to the nucleic acid sequence encoding the polypeptide variant. Such control sequences include, but are not limited to, a leader sequence, polyadenylation sequence, propeptide sequence, promoter, enhancer or upstream activating sequence, signal peptide sequence, and transcription terminator. At a minimum, the control sequences include a promoter.

A wide variety of expression control sequences may be used in the present invention. Such useful expression control sequences include the expression control sequences associated with structural genes of the foregoing expression vectors as well as any sequence known to control the expression of genes of prokaryotic or eukaryotic cells or their viruses, and various combinations thereof.

Examples of suitable control sequences for directing transcription in mammalian cells include the early and late promoters of SV40 and adenovirus, e.g. the adenovirus 2 major late promoter, the MT-1 (metallothionein gene) promoter, the human cytomegalovirus immediate-early gene promoter (CMV), the human elongation factor lα (EF-1α) promoter, the *Drosophila* minimal heat shock protein 70 promoter, the Rous Sarcoma Virus (RSV) promoter, the human ubiquitin C (UbC) promoter, the human growth hormone terminator, SV40 or adenovirus Elb region polyadenylation signals and the Kozak consensus sequence (Kozak, M. J Mol Biol 1987 Aug 20;196(4):947-50).

In order to improve expression in mammalian cells a synthetic intron may be inserted in the 5' untranslated region of the nucleotide sequence encoding the polypeptide. An example of a synthetic intron is the synthetic intron from the plasmid pCI-Neo (available from Promega Corporation, WI, USA).

Examples of suitable control sequences for directing transcription in insect cells include the polyhedrin promoter, the P10 promoter, the *Autographa californica* polyhedrosis virus basic protein promoter, the baculovirus immediate early gene 1 promoter and the baculovirus 39K delayed-early gene promoter, and the SV40 polyadenylation sequence. Examples of suitable control sequences for use in yeast host cells include the promoters of the yeast α-mating system, the yeast triose phosphate isomerase (TPI) promoter, promoters from yeast glycolytic genes or alcohol dehydrogenase genes, the ADH2-4c promoter, and the inducible GAL promoter. Examples of suitable control sequences for use in filamentous fungal host cells include the ADH3 promoter and terminator, a promoter derived from the genes encoding *Aspergillus oryzae* TAKA amylase triose phosphate isomerase or alkaline protease, an *A. niger* α-amylase, *A*. *niger* or *A*. *nidulans* glucoamylase, *A*. *nidulans* acetamidase, *Rhizomucor miehei* aspartic proteinase or lipase, the TPI1 terminator and the ADH3 terminator. Examples of suitable control sequences for use in bacterial host cells include promoters of the *lac* system, the *trp* system, the *TAC* or *TRC* system, and the major promoter regions of phage lambda.

The presence or absence of a signal peptide will, e.g., depend on the expression host cell used for the production of the polypeptide variant to be expressed (whether it is an intracellular or extracellular polypeptide) and whether it is desirable to obtain secretion. For use in filamentous fungi, the signal peptide may conveniently be derived from a gene encoding an *Aspergillus* sp. amylase or glucoamylase, a gene encoding a *Rhizomucor miehei* lipase or protease or a *Humicola lanuginosa* lipase. The signal peptide is preferably derived from a gene encoding *A*. *oryzae* TAKA amylase, *A*. *niger* neutral α-amylase, *A*. *niger* acid-stable amylase, or *A*. *niger* glucoamylase. For use in insect cells, the signal peptide may conveniently be derived from an insect gene (cf. WO 90/05783), such as the *Lepidopteran manduca sexta* adipokinetic hormone precursor, (cf. US 5,023,328), the honeybee melittin (Invitrogen), ecdysteroid UDPglucosyltransferase (egt) (Murphy et al., Protein Expression and Purification 4, 349-357 (1993) or human pancreatic lipase (hpl) (Methods in Enzymology 284, pp. 262-272, 1997). A preferred signal peptide for use in mammalian cells is that of hFVII or the murine Ig kappa light chain signal peptide (Coloma, M (1992) J. Imm. Methods 152:89-104). For use in yeast cells suitable signal peptides have been found to be the α-factor signal peptide from *S*. *cereviciae* (cf. US 4,870,008), a modified carboxypeptidase signal peptide (cf. L.A. Valls et al., Cell 48, 1987, pp. 887-897), the yeast BAR1 signal peptide (cf. WO 87/02670), the yeast aspartic protease 3 (YAP3) signal peptide (cf. M. Egel-Mitani et al., Yeast 6, 1990, pp. 127-137), and the synthetic leader sequence TA57 (WO98/32867). For use in *E. coli* cells a suitable signal peptide have been found to be the signal peptide *ompA* (EP581821).

The nucleotide sequence of the invention encoding a polypeptide variant, whether prepared by site-directed mutagenesis, synthesis, PCR or other methods, may optionally include a nucleotide sequence that encode a signal peptide. The signal peptide is present when the polypeptide variant is to be secreted from the cells in which it is expressed. Such signal peptide, if present, should be one recognized by the cell chosen for expression of the polypeptide variant. The signal peptide will typically be the one normally associated with human wild-type FVII.

Any suitable host may be used to produce the polypeptide variant, including bacteria (although not particularly preferred), fungi (including yeasts), plant, insect, mammal, or other appropriate animal cells or cell lines, as well as transgenic animals or plants. Examples of bacterial host cells include grampositive bacteria such as strains of *Bacillus,* e.g. *B. brevis* or *B*. *subtilis,* or *Streptomyces,* or gramnegative bacteria, such as strains of *E. coli.* The introduction of a vector into a bacterial host cell may, for instance, be effected by protoplast transformation (see, *e.g.,* Chang and Cohen, 1979, Molecular General Genetics 168: 111-115), using competent cells (see, *e.g.,* Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, *e.g.,* Koehler and Thorne, 1987, Journal of Bacteriology 169: 5771-5278). Examples of suitable filamentous fungal host cells include strains of *Aspergillus,* e.g. *A. oryzae, A. niger,* or *A. nidulans, Fusarium* or *Trichoderma.* Fungal cells may be transformed by a process involving protoplast formation, transformation of the protoplasts, and regeneration of the cell wall in a manner known *per se.* Suitable procedures for transformation of *Aspergillus* host cells are described in EP 238 023 and US 5,679,543. Suitable methods for transforming *Fusarium* species are described by Malardier et al., 1989, Gene 78: 147-156 and WO 96/00787. Examples of suitable yeast host cells include strains of *Saccharomyces,* e.g. *S*. *cerevisiae, Schizosaccharomyces, Kluyveromyces, Pichia,* such as *P. pastoris* or *P. methanolica, Hansenula,* such as *H. Polymorpha* or *Yarrowia.* Yeast may be transformed using the procedures described by Becker and Guarente, *In* Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153: 163; Hinnen et al., 1978, Proceedings of the National Academy of Sciences USA 75: 1920: and as disclosed by Clontech Laboratories, Inc, Palo Alto, CA, USA (in the product protocol for the Yeastmaker^{™} Yeast Transformation System Kit). Examples of suitable insect host cells include a *Lepidoptora* cell line, such as *Spodoptera frugiperda* (Sf9 or Sf21) or *Trichoplusioa ni* cells (High Five) (US 5,077,214). Transformation of insect cells and production of heterologous polypeptides therein may be performed as described by Invitrogen. Examples of suitable mammalian host cells include Chinese hamster ovary (CHO) cell lines, (e.g. CHO-K1; ATCC CCL-61), Green Monkey cell lines (COS) (e.g. COS 1 (ATCC CRL-1650), COS 7 (ATCC CRL-1651)); mouse cells (e.g. NS/O), Baby Hamster Kidney (BHK) cell lines (e.g. ATCC CRL-1632 or ATCC CCL-10), and human cells (e.g. HEK 293 (ATCC CRL-1573)), as well as plant cells in tissue culture. Additional suitable cell lines are known in the art and available from public depositories such as the American Type Culture Collection, Rockville, Maryland. Also, the mammalian cell, such as a CHO cell, may be modified to express sialyltransferase, e.g. 1,6-sialyltransferase, e.g. as described in US 5,047,335, in order to provide improved glycosylation of the polypeptide variant.

In order to increase secretion it may be of particular interest to produce the polypeptide variant of the invention together with an endoprotease, in particular a PACE (paired basic amino acid converting enzyme) (e.g. as described in US 5,986,079), such as a Kex2 endoprotease (e.g. as described in WO 00/28065).

Methods for introducing exogeneous DNA into mammalian host cells include calcium phosphate-mediated transfection, electroporation, DEAE-dextran mediated transfection, liposome-mediated transfection, viral vectors and the transfection method described by Life Technologies Ltd, Paisley, UK using Lipofectamin 2000. These methods are well known in the art and e.g. described by Ausbel et al. (eds.), 1996, Current Protocols in Molecular Biology, John Wiley & Sons, New York, USA. The cultivation of mammalian cells are conducted according to established methods, e.g. as disclosed in Animal Cell Biotechnology, Methods and Protocols, Edited by Nigel Jenkins, 1999, Human Press Inc, Totowa, New Jersey, USA and Harrison MA and Rae IF, General Techniques of Cell Culture, Cambridge University Press 1997.

In the production methods of the present invention, the cells are cultivated in a nutrient medium suitable for production of the polypeptide variant using methods known in the art. For example, the cell may be cultivated by shake flask cultivation, small-scale or large-scale fermentation (including continuous, batch, fed-batch, or solid state fermentations) in laboratory or industrial fermenters performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide variant is secreted into the nutrient medium, the polypeptide can be recovered directly from the medium. If the polypeptide variant is not secreted, it can be recovered from cell lysates.

The resulting polypeptide variant may be recovered by methods known in the art. For example, the polypeptide variant may be recovered from the nutrient medium by conventional procedures including, but not limited to, centrifugation, filtration, extraction, spray drying, evaporation, or precipitation.

The polypeptides may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), HPLC, or extraction (see, *e.g., Protein Purification,* J.-C. Janson and Lars Ryden, editors, VCH Publishers, New York, 1989).

Single chain polypeptide variants of the invention can be purified and activated to two-chain polypeptide variants by a number of methods as described in the literature (Broze and Majerus, 1980, J. Biol. Chem. 255:1242-47 and Hedner and Kisiel, 1983, J.Clin.Invest. 71:1836-41). Another method whereby single chain polypeptide variants can be purified is by incorporation of Zn ions during purification as described in US 5,700,914.

In a preferred embodiment the polypeptide variant is purified as a single chain polypeptide variant, which further is optionally PEGylated. The optionally PEGylated single chain polypeptide variant is activated by either use of an immobilized enzyme (e.g. factors IIa, IXa, Xa and XIIa) or by autoactivation using a positively charged ion exchange matrix or the like.

It is advantageous to first purify the polypeptide variant in its single chain form, then PEGylate (if desired) and lastly activate by one of the methods described above or by autoactivation as described by Pedersen et al, 1989, Biochemistry 28: 9331-36. The advantage of carrying out PEGylation before activation is that PEGylation of the new amino-terminus formed by cleavage of R 152-1153 is avoided. PEGylation of this new amino-terminus would render the molecule inactive since the formation of a hydrogen bond between D242 and the amino group of I153 is necessary for activity.

### Pharmaceutical composition of the invention and its use

In a further aspect, the present invention relates to a composition, in particular to a pharmaceutical composition, comprising a polypeptide variant of the invention and a pharmaceutically acceptable carrier or excipient.

The polypeptide variant or the pharmaceutical composition according to the invention may be used as a medicament.

Due to the high clotting efficiency, the polypeptide variant of the invention, or the pharmaceutical composition of the invention, is particular useful for the treatment of haemorrage, including uncontrollable bleeding events, such as uncontrollable bleding events in connection with trauma, thrombocytopenia, patients in anticoagulant treatment, and cirrhosis patients, such as cirrhosis patients with variceal bleeds, or other upper gastrointestinal bleedings, and in patients undergoing orthotopic liver transplantation, or liver resection (allowing for transfusion free surgery).

Trauma is defined as an injury to living tissue caused by an extrinsic agent. It is the 4^{th} leading cause of death in the US and places a large financial burden on the economy.

Trauma is classified as either blunt or penetrative. Blunt trauma results in internal compression, organ damage and internal haemorrhage whereas penetrative trauma (as the consequence of an agent penetrating the body and destroying tissue, vessels and organs) results in external haemorrhage.

Haemorrhage, as a result of trauma, can start a cascade of problems. For example physiological compensation mechanisms are initiated with initial peripheral and mesenteric vasoconstriction to shunt blood to the central circulation. If circulation is not restored, hypovolemia shock (multiple organ failure due to inadequate perfusion) ensues. Since tissues throughout the body become starved for oxygen, anaerobic metabolism begins. However, the concomitant lactic acid leads the blood pH to drop and metabolic acidosis develops. If acidosis is severe and uncorrected, the patient may develop multisystem failure and die.

Although the majority of trauma patients are hypothermic on arrival in the emergency room due to the environmental conditions at the scene, inadequate protection, intravenous fluid administration and ongoing blood loss worsen the hypothermic state. Deficiencies in coagulation factors can result from blood loss or transfusions. Meanwhile, acidosis and hypothermia interfere with blood clotting mechanisms. Thus coagulopathy develops, which in turn, may mask surgical bleeding sites and hamper the control of mechanical bleeding. Hypothermia, coagulopathy and acidosis are often characterised as the "trauma triad of death"

Trauma may be caused by several events. For example, road traffic accidents result in many different types of trauma. Whilst some road traffic accidents are likely to result in penetrative trauma, many road traffic accidents are likely to inflict blunt trauma to both head and body. However, these various types of trauma can all result in coagulopathy in the patient. Road traffic accidents are the leading cause of accidental death in the US. There are over 42,000 deaths from them in the US each year. Many trauma patients die at the location of the accident either whilst being treated by the paramedics, before they arrive or in transit to the ER.

Another example includes gunshot wounds. Gunshot wounds are traumas that can result in massive bleeding. They are penetrative and destroy tissue as the bullet passes through the body, whether it be in the torso or a limb. In the US about 40,000 people a year die from gunshot wounds

A further example includes falls. Falls result in a similar profile of trauma type to road traffic accidents. By falling onto a solid object or the ground from height can cause both penetrative and decelerative blunt trauma. In the US, falls are a common cause of accidental death, numbering about 13,000.

A still further example includes machinery accidents. A smaller number of people die in the US from machinery accident related deaths, whether struck by, or entangled in machinery. The figures are small but significant - around 2,000.

A still further example includes stab wounds. Stab wounds are penetrative injuries that can also cause massive bleeding. The organs most likely to be damaged in a stab wound are the liver, small intestine and the colon.

Cirrhosis of the liver is the terminal sequel of prolonged repeated injury to.the hepatic parenchyma. The end result is the formation of broad bands of fibrous tissue separating regenerative nodules that do not maintain the normal organization of liver lobules and thus cause deteriorated liver function. Patients have prolonged prothrombin times as a result of the depletion of vitamin K-dependent coagulation factors. Pathogenetically, liver cirrhosis should be regarded as the final common pathway of chronic liver injury, which can result from any form of intense repeated prolonged liver cell injury. Cirrhosis of the liver may be caused by direct liver injury, including chronic alcoholism, chronic viral hepatitis (types B, C, and D), and auto immune hepatitis as well as by indirect injury by way of bile duct damage, including primary biliary cirrhosis, primary sclerosing cholangitis and biliary atresia. Less common causes of cirrhosis include direct liver injury from inherited disease such as cystic fibrosis, alpha-1-antitrypsin deficiency, hemochromatosis, Wilson's disease, galactosemia, and glycogen storage disease.

Transplantation is primarily reserved for late stage cirrhotic patients, where it is the key intervention for treating the disease. To be eligible for transplantation, a patient must be classified as Child's B or C, as well as meet additional criteria for selection. Last year, in the US alone, 4,954 transplants were performed.

It has been estimated that there are 6,000 bleeding episodes associated with patients undergoing resection each year. This correlates with the reserved position of this procedure although seems slightly high in comparison with transplantation numbers.

Accurate data on the incidence of variceal bleeding is hard to obtain. The key facts known are that at the time of diagnosis, varices are present in about 60% of decompensated and 30% of compensated patients and that about 30% of these patients with varices will experience a bleed and that each episode of variceal bleeding is associated with a 30% risk of mortality.

Thrombocytopenia is caused by one of three mechanisms-decreased bone marrow production, increased splenic sequestration, or accelerated destruction of platelets. Thrombocytopenia is a risk factor for hemorrhage, and platelet transfusion reduces the incidence of bleeding. The threshold for prophylactic platelet transfusion is 10,000/µl. In patients without fever or infections, a threshold of 5000/µl may be sufficient to prevent spontaneous hemorrhage. For invasive procedures, 50,000/µl platelets is the usual target level. In patients who develop antibodies to platelets following repeated transfusions, bleeding can be extremely difficult to control.

Thus, in a further aspect the present invention relates to the use of a polypeptide variant of the invention for the manufacture of a medicament for the treatment of diseases or disorders wherein clot formation is desirable. A still further aspect of the present invention relates to a method for treating a mammal having a disease or disorder wherein clot formation is desirable, comprising administering to a mammal in need thereof an effective amount of a polypeptide variant or the pharmaceutical composition of the invention.

Examples of diseases/disorders wherein clot formation is desirable include, but is not limited to, hemorrhages, including brain hemorrhages, as well as uncontrolled bleedings, such as trauma. Further examples include patients undergoing living transplantations, patients undergoing resection, thrombocytopenic patients, cirrhotic patients, patients with variceal bleedings, haemophilia A, haemophilia B and von Willebrands disease.

The polypeptide variant of the invention is administered to patients in a therapeutically effective dose, normally one approximately paralleling that employed in therapy with rFVII such as NovoSeven®, or at lower dosage. By "therapeutically effective dose" herein is meant a dose that is sufficient to produce the desired effects in relation to the condition for which it is administered. The exact dose will depend on the circumstances, and will be ascertainable by one skilled in the art using known techniques. Normally, the dose should be capable of preventing or lessening the severity or spread of the condition or indication being treated. It will be apparent to those of skill in the art that an effective amount of a polypeptide variant or composition of the invention depends, *inter alia,* upon the disease, the dose, the administration schedule, whether the polypeptide variant or composition is administered alone or in conjunction with other therapeutic agents, the plasma half-life of the compositions, and the general health of the patient. Preferably, the polypeptide variant or composition of the invention is administered in an effective dose, in particular a dose which is sufficient to normalize the coagulation disorder.

The polypeptide variant of the invention is preferably administered in a composition including a pharmaceutically acceptable carrier or excipient. "Pharmaceutically acceptable" means a carrier or excipient that does not cause any untoward effects in patients to whom it is administered. Such pharmaceutically acceptable carriers and excipients are well known in the art (see, for example, Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000] ; and Handbook of Pharmaceutical Excipients, 3rd edition, A. Kibbe, Ed., Pharmaceutical Press [2000]).

The polypeptide variant of the invention can be formulated into pharmaceutical compositions by well-known methods. Suitable formulations are described by Remington's Pharmaceutical Sciences by E.W. Martin (Mark Publ. Co., 16th Ed., 1980).

The polypeptide variants of the invention can be used "as is" and/or in a salt form thereof. Suitable salts include, but are not limited to, salts with alkali metals or alkaline earth metals, such as sodium, potassium, calcium and magnesium, as well as e.g. zinc salts. These salts or complexes may by present as a crystalline and/or amorphous structure.

The pharmaceutical composition of the invention may be administered alone or in conjunction with other therapeutic agents. These agents may be incorporated as part of the same pharmaceutical composition or may be administered separately from the polypeptide variant of the invention, either concurrently or in accordance with another treatment schedule. In addition, the polypeptide variant or pharmaceutical composition of the invention may be used as an adjuvant to other therapies.

A "patient" for the purposes of the present invention includes both humans and other mammals. Thus, the methods are applicable to both human therapy and veterinary applications. The pharmaceutical composition comprising the polypeptide variant of the invention may be formulated in a variety of forms, e.g. as a liquid, gel, lyophilized, or as a compressed solid. The preferred form will depend upon the particular indication being treated and will be apparent to one skilled in the art.

In particular, the pharmaceutical composition comprising the polypeptide variant of the invention may be formulated in lyophilised or stable soluble form. The polypeptide variant may be lyophilised by a variety of procedures known in the art. The polypeptide variant may be in a stable soluble form by the removal or shielding of proteolytic degradation sites as described herein. The advantage of obtaining a stable soluble preparation lies in easier handling for the patient and, in the case of emergencies, quicker action, which potentially can become life saving. The preferred form will depend upon the particular indication being treated and will be apparent to one of skill in the art.

The administration of the formulations of the present invention can be performed in a variety of ways, including, but not limited to, orally, subcutaneously, intravenously, intracerebrally, intranasally, transdermally, intraperitoneally, intramuscularly, intrapulmonary, vaginally, rectally, intraocularly, or in any other acceptable manner. The formulations can be administered continuously by infusion, although bolus injection is acceptable, using techniques well known in the art, such as pumps or implantation. In some instances the formulations may be directly applied as a solution or spray.

### Parenterals

A preferred example of a pharmaceutical composition is a solution, in particular an aqueous solution, designed for parenteral administration. Although in many cases pharmaceutical solution formulations are provided in liquid form, appropriate for immediate use, such parenteral formulations may also be provided in frozen or in lyophilized form. In the former case, the composition must be thawed prior to use. The latter form is often used to enhance the stability of the active compound contained in the composition under a wider variety of storage conditions, as it is recognized by those skilled in the art that lyophilized preparations are generally more stable than their liquid counterparts. Such lyophilized preparations are reconstituted prior to use by the addition of one or more suitable pharmaceutically acceptable diluents such as sterile water for injection or sterile physiological saline solution.

In case of parenterals, they are prepared for storage as lyophilized formulations or aqueous solutions by mixing, as appropriate, the polypeptide variant having the desired degree of purity with one or more pharmaceutically acceptable carriers, excipients or stabilizers typically employed in the art (all of which are termed "excipients"), for example buffering agents, stabilizing agents, preservatives, isotonifiers, non-ionic surfactants or detergents, antioxidants and/or other miscellaneous additives.

Buffering agents help to maintain the pH in the range which approximates physiological conditions. They are typically present at a concentration ranging from about 2 mM to about 50 mM. Suitable buffering agents for use in the present invention include both organic and inorganic acids and salts thereof such as citrate buffers (e.g., monosodium citrate-disodium citrate mixture, citric acid-trisodium citrate mixture, citric acid-monosodium citrate mixture, etc.), succinate buffers (e.g., succinic acid-monosodium succinate mixture, succinic acid-sodium hydroxide mixture, succinic acid-disodium succinate mixture, etc.), tartrate buffers (e.g., tartaric acid-sodium tartrate mixture, tartaric acid-potassium tartrate mixture, tartaric acid-sodium hydroxide mixture, etc.), fumarate buffers (e.g., fumaric acid-monosodium fumarate mixture, fumaric acid-disodium fumarate mixture, monosodium fumarate-disodium fumarate mixture, etc.), gluconate buffers (e.g., gluconic acid-sodium glyconate mixture, gluconic acid-sodium hydroxide mixture, gluconic acid-potassium glyuconate mixture, etc.), oxalate buffer (e.g., oxalic acid-sodium oxalate mixture, oxalic acid-sodium hydroxide mixture, oxalic acid-potassium oxalate mixture, etc.), lactate buffers (e.g., lactic acid-sodium lactate mixture, lactic acid-sodium hydroxide mixture, lactic acid-potassium lactate mixture, etc.) and acetate buffers (e.g., acetic acid-sodium acetate mixture, acetic acid-sodium hydroxide mixture, etc.). Additional possibilities are phosphate buffers, histidine buffers and trimethylamine salts such as Tris.

Stabilizers refer to a broad category of excipients, which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, omithine, L-leucine, 2-phenylalanine, glutamic acid, threonine, etc., organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur-containing reducing agents, such as urea, glutathione, thioctic acid, sodium thioglycolate, thioglycerol, α-monothioglycerol and sodium thiosulfate; low molecular weight polypeptides (i.e. <10 residues); proteins such as human serum albumin, bovine serum albumin, gelatin or immunoglobulins; hydrophilic polymers such as polyvinylpyrrolidone; monosaccharides such as xylose, mannose, fructose and glucose; disaccharides such as lactose, maltose and sucrose; trisaccharides such as raffinose, and polysaccharides such as dextran. Stabilizers are typically present in the range of from 0.1 to 10,000 parts by weight based on the active protein weight.

Preservatives are added to retard microbial growth, and are typically added in amounts of about 0.2%-1% (w/v). Suitable preservatives for use with the present invention include phenol, benzyl alcohol, meta-cresol, methyl paraben, propyl paraben, octadecyldimethylbenzyl ammonium chloride, benzalkonium halides (e.g. benzalkonium chloride, bromide or iodide), hexamethonium chloride, alkyl parabens such as methyl or propyl paraben, catechol, resorcinol, cyclohexanol and 3-pentanol.

Isotonicifiers are added to ensure isotonicity of liquid compositions and include polyhydric sugar alcohols, preferably trihydric or higher sugar alcohols, such as glycerin, erythritol, arabitol, xylitol, sorbitol and mannitol. Polyhydric alcohols can be present in an amount between 0.1% and 25% by weight, typically 1% to 5%, taking into account the relative amounts of the other ingredients.

Non-ionic surfactants or detergents (also known as "wetting agents") may be present to help solubilizing the therapeutic agent as well as to protect the therapeutic polypeptide against agitation-induced aggregation, which also permits the formulation to be exposed to shear surface stress without causing denaturation of the polypeptide. Suitable non-ionic surfactants include polysorbates (20, 80, etc.), polyoxamers (184, 188 etc.), Pluronic® polyols, polyoxyethylene sorbitan monoethers (Tween@-20, Tween®-80, etc.).

Additional miscellaneous excipients include bulking agents or fillers (e.g. starch), chelating agents (e.g. EDTA), antioxidants (e.g., ascorbic acid, methionine, vitamin E) and cosolvents.

The active ingredient may also be entrapped in microcapsules prepared, for example, by coascervation techniques or by interfacial polymerization, for example hydroxymethylcellulose, gelatin or poly-(methylmethacylate) microcapsules, in colloidal drug delivery systems (for example liposomes, albumin microspheres, microemulsions, nanoparticles and nanocapsules) or in macroemulsions. Such techniques are disclosed in Remington's Pharmaceutical Sciences, *supra.*

Parenteral formulations to be used for *in vivo* administration must be sterile. This is readily accomplished, for example, by filtration through sterile filtration membranes.

### Sustained release preparations

Examples of sustained-release preparations include semi-permeable matrices of solid hydrophobic polymers containing the polypeptide variant, the matrices having a suitable form such as a film or microcapsules. Examples of sustained-release matrices include polyesters, hydrogels (for example, poly(2-hydroxyethyl-methacrylate) or poly(vinylalcohol)), polylactides, copolymers of L-glutamic acid and ethyl-L-glutamate, non-degradable ethylene-vinyl acetate, degradable lactic acid-glycolic acid copolymers such as the ProLease® technology or Lupron Depot® (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for long periods such as up to or over 100 days, certain hydrogels release proteins for shorter time periods. When encapsulated polypeptides remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

The invention is further described in the following non-limiting examples.

### MATERIALS AND METHODS

### Accessible Surface Area (ASA)

The computer program Access (B. Lee and F.M.Richards, J. Mol.Biol. 55: 379-400 (1971)) version 2 (© 1983 Yale University) is used to compute the accessible surface area (ASA) of the individual atoms in the structure. This method typically uses a probe-size of l.4Å and defines the Accessible Surface Area (ASA) as the area formed by the center of the probe. Prior to this calculation all water molecules and all hydrogen atoms should be removed from the coordinate set, as should other atoms not directly related to the protein.

### Fractional ASA of Side Chain

The fractional ASA of the side chain atoms is computed by division of the sum of the ASA of the atoms in the side chain with a value representing the ASA of the side chain atoms of that residue type in an extended Ala-x-Ala tripeptide (See Hubbard, Campbell & Thomton (1991) J.Mol.Bio1.220,507-530). For this example the CA atom is regarded as a part of the side chain of Glycine residues but not for the remaining residues. The following table is used as standard 100% ASA for the side chain:

| | | | | |
|---|---|---|---|---|
| Ala | 69.23 Å² | | Leu | 140.76 Å² |
| Arg | 200.35 Å² | | Lys | 162.50 Å² |
| Asn | 106.25 Å² | | Met | 156.08 Å² |
| Asp | 102.06 Å² | | Phe | 163.90 Å² |
| Cys | 96.69 Å² | | Pro | 119.65 Å² |
| Gln | 140.58 Å² | | Ser | 78.16 Å² |
| Glu | 134.61 Å² | | Thr | 101.67 Å² |
| Gly | 32.28 Å² | | Trp | 210.89 Å² |
| His | 147.00 A² | | Tyr | 176.61 Å² |
| Ile | 137.91 Å² | | Val | 114.14 Å² |

Residues not detected in the structure are defined as having 100% exposure as they are thought to reside in flexible regions. The gamma-carboxy glutamic acids at positions 6, 7, 14, 16, 19, 20, 25, 26, 29 and 35 are all defined as being 100% exposed.

### Determining Distances Between Atoms

The distance between atoms is most easily determined using molecular graphics software e.g. InsightII® v. 98.0, MSI INC.

### Active Site Region

The active site region is defined as any residues having at least one atom within 10 Å of any atom in the catalytic triad (residues H193, D242, S344).

### Determination of Tissue Factor Blinding Site

The TF binding site is defined as comprising all residues having their accessible surface area changed upon TF binding. This is determined by at least two ASA calculations; one on the isolated ligand(s) in the ligand(s)/receptor(s) complex and one on the complete ligand(s)/receptor(s) complex.

### Measurement of Reduced Sensitivity to Proteolytic Degradation

Proteolytic degradation can be measured using the assay described in US 5,580,560, Example 5, where proteolysis is autoproteolysis.

Furthermore, reduced proteolysis can be tested in *an in vivo* model using radiolabelled samples and comparing proteolysis of rhFVIIa and the polypeptide variant of the invention by withdrawing blood samples and subjecting these to SDS-PAGE and autoradiography.

Irrespectively of the assay used for determining proteolytic degradation, "reduced proteolytic degradation" is intended to mean a measurable reduction in cleavage compared to that obtained by rhFVIIa as measured by gel scanning of Coomassie stained SDS-PAGE gels, HPLC or as measured by conserved catalytic activity in comparison to wild type using the tissue factor independent activity assay decribed below.

### Determination of the Molecular Weight of Polypeptide Variants

The molecular weight of polypeptide variants is determined by either SDS-PAGE, gel filtration, Western Blots, matrix assisted laser desorption mass spectrometry or equilibrium centrifugation, e.g. SDS-PAGE according to Laemmli, U.K., Nature Vol 227 (1970), pp. 680-85.

### Determination of Tissue Factor Binding Affinity

The capacity of variants to bind to tissue factor may be evaluated using one or more of the three BIAcore® assays described in Dickinson et al. Proc. Natl. Acad. Sci. USA 1996, 93: 14379-14384; Roberge et al. Biochemistry 2001, 40: 9522-9531; and Ruf et al. Biochemistry 1999, 38(7): 1957-1966.

### Determination of TFPI Inhibition

FVII inhibition by TFPI can be monitored in the amidolytic assay described in Chang et al. Biochemistry 1999, 38: 10940-10948.

### Determination of TFPI Affinity

The capacity of variants to bind to TFPI is evaluated using one or more of the three BIAcore® assays described in Dickinson et al. Proc. Natl. Acad. Sci. USA 1996, 93: 14379-14384; Roberge et al. Biochemistry 2001, 40: 9522-9531; and Ruf et al. Biochemistry 1999, 38(7): 1957-1966.

### Determination of Phospholipid Membrane Blinding Affinity

Phospholipid membrane binding affinity may be determined as described in Nelsestuen et al., Biochemistry, 1977; 30;10819-10824 or as described in Example 1 in US 6,017,882.

### TF-independent Factor X Activation Assay

This assay has been described in detail on page 39826 in Nelsestuen et al., J Biol Chem, 2001; 276:39825-39831.

Briefly, the molecule to be assayed (either hFVIIa, rhFVIIa or the polypeptide variant of the invention in its activated form) is mixed with a source of phospholipid (preferably phosphatidylcholine and phosphatidylserine in a ratio of 8:2) and relipidated Factor X in Tris buffer containing BSA. After a specified incubation time the reaction is stopped by addition of excess EDTA. The concentration of factor Xa is then measured from absorbance change at 405 nm after addition of a chromogenic substrate (S-2222, Chromogenix). After correction from background the tissue factor independent activity of rhFVIIa (a_{wt}) is determined as the absorbance change after 10 minutes and the tissue factor independent activity of the polypeptide variant of the invention (aᵥₐᵣᵢₐₙₜ) is also determined as the absorbance change after 10 minutes. The ratio between the activity of the polypeptide variant, in its activated form, and the activity of rhFVIIa is defined as aᵥₐᵣᵢₐₙₜ/a_{wt}.

### Clotting Assay

The clotting activity of the FVIIa and variants thereof were measured in one-stage assays and the clotting times were recorded on a Thrombotrack IV coagulometer (Medinor). Factor VII-depleted human plasma (American Diagnostica) was reconstituted and equilibrated at room temperature for 15-20 minutes. 50 microliters of plasma was then transferred to the coagulometer cups.

. FVIIa and variants thereof were diluted in Glyoxaline Buffer (5.7 mM barbiturate, 4.3 mM sodium citrate, 117 mM NaCl, 1 mg/ml BSA, pH 7.35). The samples were added to the cup in 50 ul and incubated at 37°C for 2 minutes.

Thromboplastin (Medinor) was reconstituted with water and CaCl₂ was added to a final concentration of 4.5 mM. The reaction was initiated by adding 100 µl thromboplastin.

To measure the clotting activity in the absence of TF the same assay was used without addition of thromboplastin. Data was analysed using PRISM software.

### Whole Blood Assay

The clotting activity of FVIIa and variants thereof were measured in one-stage assays and the clotting times were recorded on a Thrombotrack IV coagulometer (Medinor). 100 µl of FVIIa or variants thereof were diluted in a buffer containing 10 mM glycylglycine, 50 mM NaCl, 37.5 mM CaCl₂, pH 7.35 and transferred to the reaction cup. The clotting reaction was initiated by addition of 50 µl blood containing 10% 0.13 M tri-sodium citrate as anticoagulant. Data was analysed using Excel or PRISM software.

### Amidolytic Assay

The ability of the variants to cleave small peptide substrates can be measured using the chromogenic substrate S-2288 (D-Ile-Pro-Arg-p-nitroanilide). FVIIa is diluted to about 10-90 nM in assay buffer (50 mM Na-Hepes pH 7.5, 150 mM NaCl, 5 mM CaCl₂, 0.1% BSA, IU/ml Heparin). Furthermore, soluble TF (sTF) is diluted to 50-450 nM in assay buffer. 120 µl of assay buffer is mixed with 20 µl of the FVIIa sample and 20 µl sTF. After 5 min incubation at room temperature with gentle shaking, followed by 10 min incubation at 37°C, the reaction is started by addition of the S-2288 substrate to 1 mM and the absorption at 405 nm is determined at several time points.

### ELISA Assay

FVII/FVIIa (or variant) concentrations are determined by ELISA. Wells of a microtiter plate are coated with an antibody directed against the protease domain using a solution of 2 µg/ml in PBS (100 µl per well). After overnight coating at R.T., the wells are washed 4 times with THT buffer (100 mM NaCl, 50 mM Tris-HCl pH 7.2 0.05% Tween-20). Subsequently, 200 µl of 1% Casein (diluted from 2.5% stock using 100 mM NaCl, 50 mM Tris-HCl pH 7.2) is added per well for blocking. After 1 hr incubation at R.T., the wells are emptied, and 100 µl of sample (optionally diluted in dilution buffer (THT + 0.1% Casein)) is added. After another incubation of 1 hr at room temperature, the wells are washed 4 times with THT buffer, and 100 µl of a biotin-labelled antibody directed against the EGF-like domain (1 µg/ml) is added. After another 1 hr incubation at R.T., followed by 4 more washes with THT buffer, 100 µl] of streptavidin-horse radish peroxidase (DAKO A/S, Glostrup, Denmark, 1/10000 diluted) is added. After another 1 hr incubation at R.T., followed by 4 more washes with THT buffer, 100 µl of TMB (3,3',5,5'-tetramethylbenzidine, Kem-en-Tech A/S, Denmark) is added. After 30 min incubation at R.T. in the dark, 100 µl of 1 M H₂SO₄ is added and OD₄₅₀ₙₘ is determined. A standard curve is prepared using rhFVIIa (NovoSeven®).

Alternatively, FVII/FVIIa or variants may be quantified through the Gla domain rather than through the protease domain. In this ELISA set-up, wells are coated overnight with an antibody directed against the EGF-like domain and for detection, a calcium-dependent biotin-labelled monoclonal anti-Gla domain antibody is used (2 µg/ml, 100 µl per well). In this set-up, 5 mM CaCl₂ is added to the THT and dilution buffers.

### EXAMPLES

### Example 1

The X-ray structure of hFVIIa in complex with soluble tissue factor by Banner et al., J Mol Biol, 1996; 285:2089 is used for this example. It is noted that the numbering of residues in the reference does not follow the sequence. Here we have used the sequential numbering according to SEQ ID NO:1. The gamma-carboxy glutamic acids at positions 6, 7, 14, 16, 19, 20, 25, 26, 29 and 35 are all here named Glu (three letter abbreviation) or E (one letter abbreviation). Residues 143-152 are not present in the structure.

### Surface Exposure

Performing fractional ASA calculations on FVII fragments alone combined with the definition of accessibilities of non standard and/or missing residues described in the methods resulted in the following residues having more than 25% of their side chain exposed to the surface: A1, N2, A3, F4, L5, E6, E7, L8, R9, P10, S12, L13, E14, E16, K18, E19, E20, Q21, S23, F24, E25, E26, R28, E29, F31, K32, D33, A34, E35, R36, K38, L39, W41, I42, S43, S45, G47, D48, Q49, A51, S52, S53, Q56, G58, S60, K62, D63, Q64, L65, Q66, S67, I69, F71, L73, P74, A75, E77, G78, R79, E82, T83, H84, K85, D86, D87, Q88, L89, 90, V92, N93, E94, G97, E99, S103, D104, H105, T106, G107, T108, K109, S111, R113, E116, G117, S119, L120, L121, A122, D123, G124, V125, S126, T128, P129, T130, V131, E132,1140, L141, E142, K143, R144, N145, A146, S147, K148, P149, Q150, G151, R152, G155, K157, V158, P160, K161, E163, L171, N173, G174, A175, N184, T185, I186, H193, K197, K199, N200, R202, N203,I205, S214, E215, H216, D217, G218, D219, S222, R224, S232, T233, V235, P236, G237, T238, T239, N240, H249, Q250, P251, V253, T255, D256, E265, R266, T267, E270, R271, F275, V276, R277, F278, L280, L287, L288, D289, R290, G291, A292, T293, L295, E296, N301, M306, T307, Q308, D309, L311, Q312, Q313, R315, K316, V317, G318, D319, S320, P321, N322, T324, E325, Y326, Y332, S333, D334, S336, K337, K341, G342, H351, R353, G354, Q366, G367, T370, V371, G372, R379, E385, Q388, K389, R392, S393, E394, P395, R396, P397, G398, V399, L400, L401, R402, P404 and P406.

The following residues had more than 50% of their side chain exposed to the surface: A1, A3, F4, L5, E6, E7, L8, R9, P10, E14, E16, K18, E19, E20, Q21, S23, E25, E26, E29, K32, A34, E35, R36, K38, L39, I42, S43, G47, D48, A51, S52, S53, Q56, G58, S60, K62, L65, Q66, S67, I69, F71, L73, P74, A75, E77, G78, R79, E82, H84, K85, D86, D87, Q88, L89, I90, V92, N93, E94, G97, T106, G107, T108, K109, S111, E116, S119, L121, A122, D123, G124, V131, E132, L141, E142, K143, R144, N145, A146, S147, K148, P149, Q150, G151, R152, G155, K157, P160, N173, G174, A175, K197, K199, N200, R202, S214, E215, H216, G218, R224, V235, P236, G237, T238, H249, Q250, V253, D256, T267, F275, R277, F278, L288, D289, R290, G291, A292, T293, L295, N301, M306, Q308, D309, L311, Q312, Q313, R315, K316, G318, D319, N322, E325, D334, K341, G354, G367, V371, E385, K389, R392, E394, R396, P397, G398, R402, P404 and P406.

### Tissue Factor Binding Site

Performing ASA calculations the following residues in human FVII change their ASA in the complex. These residues were defined as constituting the tissue factor binding site: L13, K18, F31, E35, R36, L39, F40, I42, S43, S60, K62, D63, Q64, L65, I69, C70, F71, C72, L73, P74, F76, E77, G78, R79, E82, K85, Q88, I90, V92, N93, E94, R271, A274, F275, V276, R277, F278, R304, L305, M306, T307, Q308, D309, Q312, Q313, E325 and R379.

### Active Site Region

The active site region is defined as any residue having at least one atom within a distance of 10 Å from any atom in the catalytic triad (residues H193, D242, S344): I153, Q167, V168, L169, L170, L171, Q176, L177, C178, G179, G180, T181, V188, V189, S190, A191, A192, H193, C194, F195, D196, K197, I198, W201, V228, I229, I230, P231, S232, T233, Y234, V235, P236, G237, T238, T239, N240, H241, D242, I243, A244, L245, L246, V281, S282, G283, W284, G285, Q286, T293, T324, E325, Y326, M327, F328, D338, S339, C340, K341, G342, D343, S344, G345, G346, P347, H348, L358, T359, G360, I361, V362, S363, W364, G365, C368, V376, Y377, T378, R379, V380, Q382, Y383, W386, L387, L400 and F405.

### The Ridge of the Active Site Binding Cleft

The ridge of the active site binding cleft region was defined by visual inspection of the FVIIa structure 1FAK.pdb as: N173, A175, K199, N200, N203, D289, R290, G291, A292, P321 and T370.

### Example 2

### Design of an expression cassette for expression of rhFVII in mammalian cells

The DNA sequence shown in SEQ ID NO:2, encompassing the short form of the full length cDNA encoding human blood coagulation factor VII with its native short signal peptide (Hagen et al., 1986. PNAS 83:2412), was synthesized in order to facilitate high expression in mammalian cells. First the ATG start codon context was modified according to the Kozak consensus sequence (Kozak, M. J Mol Biol 1987 Aug 20;196(4):947-50), so that there is a perfect match to the consensus sequence upstream of the ATG start codon. Secondly the open reading frame of the native human blood coagulation factor cDNA was modified by making a bias in the codon usage towards the codons frequently used in highly expressed human genes. Further, two translational stop codons were inserted at the end of the open reading frame in order to facilitate efficient translational stop. The fully synthetic and expression optimized human FVII gene was assembled from 70-mer DNA oligonucleotides and finally amplified using end primers inserting *Bam*HI and *Hind*III sites at the 5' and 3' ends respectively using standard PCR techniques.

A vector for the cloning of the generated PCR product encompassing the expression cassette for factor VII was prepared by cloning the intron from pCINeo (Promega). The synthetic intron from pCI-Neo was amplified using standard PCR conditions and the primers:
CBProFpr174: AGCTGGCTAGCCACTGGGCAGGTAAGTATCA (SEQ ID NO:3) and
CBProFpr175: TGGCGGGATCCTTAAGAGCTGTAATTGAACT (SEQ ID NO:4)
resulting in a 332 bp PCR fragment. The fragment was cut with *Nhe*I and *Bam*HI before cloning into pCDNA3.1/HygR (obtained from Invitrogen) resulting in PF#34.

The expression cassette for human factor VII was cloned between the *Bam*HI and *Hind*III sites of PF#34, resulting in plasmid PF#226.

In order to allow for cloning of FVII-variant genes between the *Nhe*I and *Pme*I sites of the UCOE based expression-plasmid CET720 a derivative of PF#226 lacking the *Nhe*I site was created. Using PF#226 as template a DNA fragment was made by PCR using the primers CBProFpr219 (SEQ ID NO:5) and CBProFpr499 (SEQ ID NO:6). This fragment was cut with *Xba*I and *Xho*I and cloned between the *Xba*I and *Xho*I sites of PF#226 resulting in the plasmid PF#444. PF#444-derivative plasmids encoding variants of the invention were constructed by standard PCR-based site-directed mutagenesis using PF#444 as template and custom-synthesized primers. Specific CET720 based expression plasmids were generated by excision of the variant gene from the corresponding PF444-derivative by *Nhe*I and *Pme*I and then cloned into *Nhe*I and *Pme*I cut CET720. For example the CET720-derivative pB0088 encoding the FVII S43Q variant was made by excision of the variant gene by *Nhe*I and *Pme*I from pB0075 and cloned into *Nhe*I and *Pme*I cut CET720.

### Example 3

### Construction of expression vectors encoding polypeptide variants of the invention

The following primers were used pair vice for the primary PCRs:

| [L39H]rhFVII | |
|---|---|
| CBProFpr526: | GCTGAGCGGACCAAACACTTTTGGATTAGC (SEQ ID NO:7 - direct primer) |
| CBProFpr527: | GCTAATCCAAAAGTGTTTGGTCCGCTCAGC (SEQ ID NO:8 - reverse primer) |

| [I42R]rhFVII | |
|---|---|
| CBProFpr530: | CCAAACTGTTTTGGCGCAGCTATAGCGATG (SEQ ID NO:9 - direct primer) |
| CBProFpr531: | CATCGCTATAGCTGCGCCAAAACAGTTTGG (SEQ ID NO:10 - reverse primer) |

| [S43Q]rhFVII | |
|---|---|
| CBProFpr534: | AACTGTTTTGGATTCAGTATAGCGATGGCG (SEQ ID NO:11 - direct primer) |
| CBProFpr535: | CGCCATCGCTATACTGAATCCAAAACAGTT (SEQ ID NO:12 - reverse primer) |

| [K62E]rhFVII | |
|---|---|
| CBProFpr536: | AACGGGGGCTCCTGCGAGGACCAGCTGCAG (SEQ ID NO:13 - direct primer) |
| CBProFpr537: | CTGCAGCTGGTCCTCGCAGGAGCCCCCGTT (SEQ ID NO:14 - reverse primer) |

| [K62R]rhFVII | |
|---|---|
| CBProFpr538: | GGGGGCTCCTGCCGCGACCAGCTGCAGAGC (SEQ NO:15 - direct primer) |
| CBProFpr539: | GCTCTGCAGCTGGTCGCGGCAGGAGCCCCC (SEQ ID NO:16 - reverse primer) |

| [F71E]rhFVII | |
|---|---|
| CBProFpr540: | GAGCTATATCTGCGAGTGCCTGCCTGCCTT (SEQ ID NO:17 - direct primer) |
| CBProFpr541: | AAGGCAGGCAGGCACTCGCAGATATAGCTC (SEQ DI NO:18 - reverse primer) |

| [E82O]rhFVII | |
|---|---|
| CBProFpr542: | GGGGCGCAATTGCCAGACCCATAAGGATGA (SEQ NO:19 - direct primer) |
| CBProFpr543: | TCATCCTTATGGGTCTGGCAATTGCGCCCC (SEQ ID NO:20 - reverse primer) |

| [L39E]rhFVII | |
|---|---|
| LoB069: | GAGCGGACCAAAGAGTTTTGGATTAGC (SEQ NO:21 - direct primer) |
| LoB070: | GCTAATCCAAAACTCTTTGGTCCGCTG (SEQ DOD NO:22 - reverse primer) |

| [L39Q]rhFVII | |
|---|---|
| LoB071: | GAGCGGACCAAACAGTTTTGGATTAGC (SEQ ID NO 23 - direct primer) |
| LoB072: | GCTAATCCAAAACTGTTTGGTCCGCTC (SEQ ID NO:24 - reverse primer) |

| [L65Q]rhFVII | |
|---|---|
| LoB075: | CTGCAAAGACCAGCAGCAGAGCTATATCTGC (SEQ ID NO:25 - direct primer) |
| LoB076: | GCAGATATAGCTCTGCTGCTGGTCTTTGCAG (SEQ ID NO:26 - reverse primer) |

| [L65S]rhFVII | |
|---|---|
| LoB077: | CTGCAAAGACCAGTCCCAGAGCTATATCTGC (SEQ ID NO:27 - direct primer) |
| LoB078: | GCAGATATAGCTCTGGGACTGGTCTTTGCAG (SEQ ID NO:28 - reverse primer) |

| [F71D]rhFVII | |
|---|---|
| LoB079: | CAGAGCTATATCTGCGACTGCCTGCCTGCC (SEQ ID NO:29 - direct primer) |
| LoB080: | GGCAGGCAGGCAGTCGCAGATATAGCTCTG (SEQ ID NO:30 - reverse primer) |

| [F71Y]rhFVII | |
|---|---|
| LoB081: | CAGAGCTATATCTGCTACTGCCTGCCTGC (SEQ ID NO:31 - direct primer) |
| LoB082: | GCAGGCAGGCAGTAGCAGATATAGCTCTG (SEQ ID NO: 32 - reverse primer) |

| [K62E+L65Q]rhFVII | |
|---|---|
| CBProFpr703: | GAACGGGGGCTCCTGCGAGGACCAGCAGCAGAGCTATATCTGC (SEQ NO:33 - direct primer) |
| CBProFpr704: | GCAGATATAGCTCTGCTGCTGGTCCTCGCAGGAGCCCCCGTTC (SEQ ID NO:34 - reverse primer) |

### Example 4

### Expression of FVII or FVII variants in CHO K1 cells

The cell line CHO K1 (ATCC # CCL-61) was seeded at 50% confluence in T-25 flasks using MEMα, 10% FCS (GibcoBRL Cat # 10091), P/S and 5 µg/ml phylloquinone and allowed to grow until confluent. The confluent mono cell layer was transfected with 5 *µ*/*g* of the relevant plasmid described above using the Lipofectamine 2000 transfection agent (Life technologies) according to the manufacturer's instructions. Twenty four hours post transfection a sample was drawn and quantified using *e.g*. an ELISA recognizing the EGF1 domain of human factor VII. At this time point relevant selection (e.g. Hygromycin B) may be applied to the cells with the purpose of generating a pool of stable transfectants. When using CHO K1 cells and the Hygromycin B resistance gene as selectable marker on the plasmid, this is usually achieved within one week.

### Example 5

### Generation of CHO-K1 cells stably expressing polypeptide variants

A vial of CHO-K1 transfectant pool was thawed and the cells seeded in a 175 cm² tissue flask containing 25 ml of MEMα, 10% FCS, phylloquinone (5 µg/ml), 100 U/1 penicillin, 100µg/l streptomycin and grown for 24 hours. The cells were harvested, diluted and plated in 96 well microtiter plates at a cell density of ½-1 cell/well. After a week of growth, colonies of 20-100 cells were present in the wells and those wells containing only one colony were labelled. After a further two weeks, the media in all wells containing only one colony was substituted with 200 µl fresh medium. After 24 hours, a medium sample was withdrawn and analysed by e.g. ELISA. High producing clones were selected and used to produce FVII or variants thereof.

### Example 6

### Small-scale purification of polypeptide variants and subsequent activation with prothrombin activator

FVII and variants thereof were purified and activated as follows. The procedure was performed at 4°C. 100 mM NaCl and 10 mM CaCl₂ wa added to 1200 ml harvested culture media followed by pH adjustment to 7.5 and sterile filtering. An affinity column was prepared by coupling a monoclonal calcium-dependent antiGla-domain antibody to CNBr-activated Sepharose FF using about 5.5 mg antibody coupled per ml resin. The prepared culture media was applied overnight to a 2 ml monoclonal antibody affinity column pre-equilibrated with 10 mM Tris, 100 mM NaCl, 35 mM CaCl₂, pH 7.5. The monoclonal antibody affinity matrix with bound FVII was subsequently unpacked by transferring the column material from the column to and empty tube.

FVII or variants thereof were activated to FVIIa by incubation with prothrombin activator from *Oxyuranus scutellatus* (OSII) while still being bound to the affinity matrix. FVIIa was recovered by repacking the column material followed by elution using 10 mM Tris, 25 mM NaCl, 5 mM EDTA, pH 8.6.

The eluate from the first chromatographic step was loaded directly onto a second and final chromatographic column, which consisted of a POROS HQ50 column pre-equilibrated with 10 mM Tris, 25 mM NaCl, 5 mM EDTA, pH 8.6. FVIIa was eluted from the POROS HQ50 column using 10 mM Tris, 25 mM NaCl, 35 mM CaCl₂, pH 7.5 after washing the column with 10 mM Tris, 25 mM NaCl, pH 8.6. FVIIa eluted from the POROS HQ50 column was stored at -80°C without further modification.

### Example 7

### Large-scale purification of polypeptide variants and subsequent activation

FVII and FVII variants are purified as follows. The procedure is performed at 4°C. The harvested culture media from large-scale production is ultrafiltered using a Millipore TFF system with 30 KDa cut-off Pellicon membranes. After concentration of the medium, citrate is added to 5 mM and the pH is adjusted to 8.6. If necessary, the conductivity is lowered to below 10 mS/cm. Subsequently, the sample is applied to a Q-sepharose FF column, equilibrated with 50 mM NaCl, 10 mM Tris pH 8.6. After washing the column with 100 mM NaCl, 10 mM Tris pH 8.6, followed by 150 mM NaCl, 10 mM Tris pH 8.6, FVII is eluted using 10 mM Tris, 25 mM NaCl, 35 mM CaCl₂, pH 8.6.

For the second chromatographic step, an affinity column is prepared by coupling of a monoclonal Calcium-dependent antiGla-domain antibody to CNBr-activated Sepharose FF. About 5.5 mg antibody is coupled per ml resin. The column is equilibrated with 10 mM Tris, 100 mM NaCl, 35 mM CaCl₂, pH 7.5. NaCl is added to the sample to a concentration of 100 mM NaCl and the pH is adjusted to 7.4 -7.6. After O/N application of the sample, the column is washed with 100 mM NaCl, 35 mM CaCl₂, 10 mM Tris pH 7.5, and the FVII protein is eluted with 100 mM NaCl, 50 mM citrate, 75 mM Tris pH 7.5.

For the third chromatographic, the conductivity of the sample is lowered to below 10 mS/cm, if necessary, and the pH is adjusted to 8.6. The sample is then applied to a Q-sepharose column (equilibrated with 50 mM NaCl, 10 mM Tris pH 8.6) at a density around 3-5 mg protein per ml gel to obtain efficient activation. After application, the column is washed with 50 mM NaCl, 10 mM Tris pH 8.6 for about 4 hours with a flow of 3-4 column volumes (cv) per hour. The FVII protein is eluted using a gradient of 0-100% of 500 mM NaCl, 10 mM Tris pH 8.6 over 40 cv. FVII containing fractions are pooled.

For the final chromatographic step, the conductivity is lowered to below 10 mS/cm. Subsequently, the sample is applied to a Q-sepharose column (equilibrated with 140 mM NaCl, 10 mM glycylglycine pH 8.6) at a concentration of 3-5 mg protein per ml gel. The column is then washed with 140 mM NaCl, 10 mM glycylglycine pH 8.6 and FVII is eluted with 140 mM NaCl, 15 mM CaCl₂, 10 mM glycylglycine pH 8.6. The eluate is diluted to 10 mM CaCl₂ and the pH is adjusted 6.8-7.2. Finally, Tween-80 is added to 0.01% and the pH is adjusted to 5.5 for storage at -80°C.

### Example 8

### Experimental results

Subjecting the variants of the invention (purified as described in Example 6 above) to the "Whole Blood Assay" revealed that the variants exhibit a significantly increased clotting activity (or reduced clotting time) as compared to rhFVIIa. The experimental results are shown in Figs. 1-3.

## Claims

1. A Factor VII or Factor VIIla polypeptide variant, said variant having an amino acid sequence which differs from human Factor VII or human Factor VIIa having the amino acid sequence shown in SEQ ID NO:1 in no more than 15 amino acid residues, wherein said variant sequence comprises the substitution L65Q and possesses increased clotting activity or reduced clotting time as compared to hFVIIa or rhFVIIa.

2. The variant according to claim 1, further comprising at least one substitution selected from the group consisting of L39E, L39Q, L39H, 142R, S43H, S43Q, K62E, K62R, L65S, F71 D, F71 Y, F71E. F71 Q, F71 N, E82Q, E82N, E82K and F275H.

3. The variant according to claim 2, wherein said at least one substitution is F71Y, K62E or S43Q.

4. The variant according to any of claims 1-3, wherein said variant comprises at least one amino acid substitution in the Gla domain.

5. The variant according to claim 4, wherein said at least one substitution in the Gla domain comprises a substitution in at least one position selected from the group consisting of P10, K32, D33 and A34.

6. The variant according to claim 5, wherein said variant further comprises an insertion of at least one amino acid residue between position A3 and F4.

7. The variant according to any of the preceding claims, wherein at least one amino acid residue comprising an attachment group for a non-polypeptide moiety has been introduced in a position located outside the Gla domain.

8. The variant according to claim 7, wherein said attachment group is an *in vivo* glycosylation site.

9. The variant according to claim 8, wherein said *in vivo* glycosylation site is an N-glycosylation site introduced by a substitution selected from the group consisting of A51 N, G58N, G48N+S60T, T106N, K109N, G124N, K143N+N145T, A175T, 1205S, 1205T, V253N, T267N, T267N+S269T, S314N+K316S, S314N+K316T, R315N+V317S, R315N+V317T, K316N+G318S, K316N+G318T, G318N, and D334N.

10. The variant according to any of the preceding claims, wherein said variant further comprises at least one substitution in a position selected from the group consisting of 157, 158, 296, 298, 305, 334, 336, 337 and 374.

11. The variant according to any of the preceding claims, wherein said variant further comprises at least one substitution selected from the group consisting of K341 Q, D196K, D196N, G237L and G237GAA.

12. The variant according to any of the preceding claims, wherein said variant is in its activated form.

13. A nucleotide sequence encoding a variant as defined in any of claims 1-12.

14. An expression vector comprising a nucleotide sequence as defined in claim 13.

15. A host cell comprising a nucleotide sequence as defined in claim 13 or an expression vector as defined in claim 14.

16. The host cell according to claim 15, wherein said host cell is a gamma-carboxylating cell capable of *in vivo* glycosylation.

17. A pharmaceutical composition comprising a variant as defined in any of claims 1-12, and a pharmaceutically acceptable carrier or excipient.

18. A variant as defined in any of claims 1-12, or a pharmaceutical composition as defined in claim 17 for use as a medicament.

19. Use of a variant as defined in any of claims 1-12 for the manufacture of a medicament for the treatment of a disease or a disorder wherein clot formation is desirable.

## Patentansprüche

1. Eine Variante von Faktor VII- oder Faktor VII a-Polypeptid, wobei die Variante eine Aminosäuresequenz hat, die sich von humanem Faktor VII oder humanem Faktor VII a mit der in SEQ ID NO:1 gezeigten Anminosäuresequenz in nicht mehr als 15 Aminosäureresten unterscheidet, wobei die Variantensequenz die Substitution L65Q umfasst und eine erhöhte Gerinnungsaktivität oder eine verringerte Gerinnungszeit im Vergleich zu hFVIIa oder rhFVIIa besitzt.

2. Variante nach Anspruch 1, weiterhin umfassend wenigstens eine Substitution, ausgewählt aus der Gruppe, bestehend aus L39E, L39Q, L39H, I42R, S43H, S43Q, K62E, K62R, L65S, F71D, F71Y, F71E, F71Q, F71N, E82Q, E82N, E82K und F275H.

3. Variante nach Anspruch 2, wobei die wenigstens eine Substitution F71Y, K62E oder S43Q ist.

4. Variante nach einem der Ansprüche 1-3, wobei die Variante wenigstens eine Aminosäuresubstitution in der Gla-Domäne umfasst.

5. Variante nach Anspruch 4, wobei die wenigstens eine Substitution in der Gla-Domäne eine Substitution in wenigstens einer Position umfasst, ausgewählt aus der Gruppe, bestehend aus P10, K32, D33 und A34.

6. Variante nach Anspruch 5, wobei die Variante weiterhin eine Insertion von wenigstens einem Aminosäurerest zwischen Position A3 und F4 umfasst.

7. Variante nach einem der vorangehenden Ansprüche, wobei wenigstens ein Aminosäurerest, umfassend eine Anhängungsgruppe für eine Nicht-Polypeptid-Gruppe, in einer Position eingeführt worden ist, die sich außerhalb der Gla-Domäne befindet.

8. Variante nach Anspruch 7, wobei die Anhängungsgruppe eine In-vivo-Glycosylierungsstelle ist.

9. Variante nach Anspruch 8, wobei die In-vivo-Glycosylierungsstelle eine N-Glycosylierungsstelle ist, eingeführt durch eine Substitution, ausgewählt aus der Gruppe, bestehend aus A51N, G58N, G48N+S60T, T106N, K109N, G124N, K143N+N145T, A175T, I205S, 1205T, V253N, T267N, T267N+S269T, S314N+K316S, S314N+K316T, R315N+V317S, R315N+V317T, K316N+G318S, K316N+G318T, G318N, und D334N.

10. Variante nach einem der vorangehenden Anspruche, wobei die Variante weiterhin wenigstens eine Substitution in einer Position umfasst, ausgewählt aus der Gruppe, bestehend aus 157, 158, 296, 298, 305, 334, 336, 337 und 374.

11. Variante nach einem der vorangehenden Ansprüche, wobei die Variante weiterhin wenigstens eine Substitution umfasst, ausgewählt aus der Gruppe, bestehend aus K341Q, D196K, D196N, G237L und G237GAA.

12. Variante nach einem der vorangehenden Ansprüche, wobei die Variante in ihrer aktivierten Form ist.

13. Nukleotidsequenz, die für eine Variante, wie in einem der Ansprüche 1-12 definiert, kodiert.

14. Expressionsvektor, umfassend eine Nukleotidsequenz, wie in Anspruch 13 definiert.

15. Wirtszelle, umfassend eine Nukleotidsequenz, wie in Anspruch 13 definiert, oder einen Expressionsvektor, wie in Anspruch 14 definiert.

16. Wirtszelle nach Anspruch 15, wobei die Wirtszelle eine Gamma-carboxylierende Zelle ist, die zur In-vivo-Glycosylierung in der Lage ist.

17. Pharmazeutische Zusammensetzung, umfassend eine Variante, wie in einem der Ansprüche 1-12 definiert, und einen pharmazeutisch akzeptablen Träger oder Bindemittel.

18. Variante, wie in einem der Ansprüche 1-12 definiert, oder eine pharmazeutische Zusammensetzung, wie in Anspruch 17 definiert, zur Verwendung als ein Medikament.

19. Verwendung einer Variante, wie in einem der Ansprüche 1-12 definiert, zur Herstellung eines Medikaments zur Behandlung einer Krankheit oder Störung, bei der Gerinnungsbildung erwünscht ist.

## Revendications

1. Variant de polypeptide de Facteur VII ou de Facteur VIIa, ledit variant ayant une séquence d'acides aminés qui diffère du Facteur VII humain ou du Facteur VIIa humain ayant la séquence d'acides aminés indiquée dans SEQ ID NO:1 dans pas plus que 15 résidus d'acides aminés, où ladite séquence de variants comprend la substitution de L65Q et possède une activité de coagulation accrue ou un temps de coagulation réduit en comparaison avec hFVIIa ou rhFVIIa.

2. Variant selon la revendication 1, comprenant en outre au moins une substitution sélectionnée dans le groupe consistant en L39E, L39Q, L39H, 142R, S43H, S43Q, K62E, K62R, L65S, F71D, F71Y, F71E, F71Q, F71N, E82Q, E82N, E82K et F275H.

3. Variant selon la revendication 2, où ladite au moins une substitution est F71Y, K62E ou S43Q.

4. Variant selon l'une quelconque des revendications 1 à 3, où ledit variant comprend au moins une substitution d'acide aminé dans le domaine Gla.

5. Variant selon la revendication 4, où ladite au moins une substitution dans le domaine Gla comprend une substitution dans au moins une position sélectionnée dans le groupe consistant en P10, K32, D33 et A34.

6. Variant selon la revendication 5, où ledit variant comprend en outre une insertion d'au moins un résidu d'acide aminé entre les positions A3 et F4.

7. Variant selon l'une quelconque des revendications précédentes, où au moins un résidu d'acide aminé comprenant un groupe d'attachement d'un fragment non-polypeptide a été introduit dans une position située à l'extérieur du domaine Gla.

8. Variant selon la revendication 7, où ledit groupe d'attachement est un site de glycosylation in vivo.

9. Variant selon la revendication 8, où ledit site de glycosylation in vivo est un site de N-glycosylation introduit par une substitution sélectionnée dans le groupe consistant en A51N, G58N, G48N+S60T, T106N, K109N, G124N, K143N+N145T, A175T, 1205S, 1205T, V253N, T267N, T267N+S269T, S314N+K316S, S314N+K316T, R315N+V317S, R315N+V317T, K316N+G318S, K316N+G318T, G318N et D334N.

10. Variant selon l'une quelconque des revendications précédentes, où ledit variant comprend en outre au moins une substitution dans une position sélectionnée dans le groupe consistant en 157, 158, 296, 298, 305, 334, 336, 337 et 374.

11. Variant selon l'une quelconque des revendications précédentes, où ledit variant comprend en outre au moins une substitution sélectionnée dans le groupe consistant en K341Q, D196K, D196N, G237L et G237GAA.

12. Variant selon l'une quelconque des revendications précédentes, où ledit variant se trouve dans sa forme activée.

13. Séquence de nucléotides codant pour un variant tel que défini dans l'une quelconque des revendications 1 à 12.

14. Vecteur d'expression comprenant une séquence de nucléotides telle que définie dans la revendication 13.

15. Cellule hôte comprenant une séquence de nucléotides telle que définie dans la revendication 13 ou un vecteur d'expression tel que défini dans la revendication 14.

16. Cellule hôte selon la revendication 15, où ladite cellule hôte est une cellule de gamma-carboxylation apte d'une glycosylation *in vivo.*

17. Composition pharmaceutique comprenant un variant tel que défini dans l'une quelconque des revendications 1 à 12 et un support ou excipient pharmaceutiquement acceptable.

18. Variant selon l'une quelconque des revendications 1 à 12 ou une composition pharmaceutique telle que définie dans la revendication 17, pour utilisation comme médicament.

19. Utilisation d'un variant tel que défini dans l'une quelconque des revendications 1 à 12 pour la fabrication d'un médicament pour le traitement d'une maladie ou d'un trouble où la formation de caillots est souhaitable.


### SEQUENCE LISTING

<110> Maxygen ApS; Maxygen Holdings
<120> FVII or FVIIa variants having increased clotting activity
<130> 0267wo310
<140>
   <141>
<160> 34
<170> PatentIn Ver. 2.1
<210> 1
   <211> 406
   <212> PRT
   <213> Homo sapiens
<400> 1
<210> 2
   <211> 1338
   <212> DNA
   <213> Homo sapiens
<220>
   <221> CDS
   <222> (115)..(1335)
<400> 2
   atggtcagcc aggccctccg cctcctgtgc ctgctcctgg ggctgcaggg ctgcctggct 60
   gccgtcttcg tcacccagga ggaagcccat ggcgtcctgc atcgccggcg ccgg gcc    117
   aat gcc ttt ctg gaa gag ctc cgc cct ggc tcc ctg gaa cgc gaa tgc    165
   aaa gag gaa cag tgc agc ttt gag gaa gcc cgg gag att ttc aaa gac    213
   gct gag cgg acc aaa ctg ttt tgg att agc tat agc gat ggc gat cag    261
   tgc gcc tcc agc cct tgc cag aac ggg ggc tcc tgc aaa gac cag ctg    309
   cag agc tat atc tgc ttc tgc ctg cct gcc ttt gag ggg cgc aat tgc    357
   gaa acc cat aag gat gac cag ctg att tgc gtc aac gaa aac ggg ggc    405
   tgc gag cag tac tgc agc gat cac acg ggc acg aag cgg agc tgc cgc    453
   tgc cac gaa ggc tat agc ctc ctg gct gac ggg gtg tcc tgc acg ccc    501
   acg gtg gaa tac cct tgc ggg aag att ccc att cta gaa aag cgg aac     549
   gct agc aaa ccc cag ggc cgg atc gtc ggc ggg aag gtc tgc cct aag     597
   ggg gag tgc ccc tgg cag gtc ctg ctc ctg gtc aac ggg gcc cag ctg     645
   tgc ggc ggg acc ctc atc aat acc att tgg gtc gtg tcc gcc gct cac     693
   tgc ttc gat aag att aag aat tgg cgg aac ctc atc gct gtg ctc ggc     741
   gaa cac gat ctg tcc gag cat gac ggg gac gaa cag tcc cgc cgg gtg     789
   gct cag gtc atc att ccc tcc acc tat gtg cct ggc acg acc aat cac     837
   gat atc gct ctg ctc cgc ctc cac cag ccc gtc gtg ctc acc gat cac     885
   gtc gtg cct ctg tgc ctg cct gag cgg acc ttt agc gaa cgc acg ctg     933
   gct ttc gtc cgc ttt agc ctc gtg tcc ggc tgg ggc cag ctg ctc gac     981
   cgg ggc gct acc gct ctc gag ctg atg gtg ctc aac gtc ccc cgg ctg     1029
   atg acc cag gac tgc ctg cag cag tcc cgc aaa gtg ggg gac tcc ccc     1077
   aat atc acg gag tat atg ttt tgc gct ggc tat agc gat ggc tcc aag     1125
   gat agc tgc aag ggg gac tcc ggc ggg ccc cat gcc acg cac tat cgc     1173
   ggg acc tgg tac ctc acc ggg atc gtc agc tgg ggc cag ggc tgc gcc     1221
   acg gtg ggg cac ttt ggc gtc tac acg cgc gtc agc cag tac att gag    1269
   tgg ctg cag aag ctc atg cgg agc gaa ccc cgg ccc ggg gtg ctc ctg    1317
   cgg gcc cct ttc cct tga taa                                        1338
<210> 3
   <211> 31
   <212> DNA
   <213> Artificial Sequence
<220>
   <223> Description of Artificial Sequence: Primer
<400> 3
   agctggctag ccactgggca ggtaagtatc a 31
<210> 4
   <211> 31
   <212> DNA
   <213> Artificial Sequence
<220>
   <223> Description of Artificial Sequence: Primer
<400> 4
   tggcgggatc cttaagagct gtaattgaac t 31
<210> 5
   <211> 23
   <212> DNA
   <213> Artificial Sequence
<220>
   <223> Description of Artificial Sequence: Primer
<400> 5
   tcagctcgag agcggtagcg ccc 23
<210> 6
   <211> 28
   <212> DNA
   <213> Artificial Sequence
<220>
   <223> Description of Artificial Sequence: Primer
<400> 6
   cccattctag aaaagcggaa cgccagcaaa ccccaggg 28
<210> 7
   <211> 30
   <212> DNA
   <213> Artificial Sequence
<220>
   <223> Description of Artificial Sequence: Primer
<400> 7
   gctgagcgga ccaaacactt ttggattagc 30
<210> 8
   <211> 30
   <212> DNA
   <213> Artificial Sequence
<220>
   <223> Description of Artificial Sequence: Primer
<400> 8
   gctaatccaa aagtgtttgg tccgctcagc 30
<210> 9
   <211> 30
   <212> DNA
   <213> Artificial Sequence
<220>
   <223> Description of Artificial Sequence: Primer
<400> 9
   ccaaactgtt ttggcgcagc tatagcgatg 30
<210> 10
   <211> 30
   <212> DNA
   <213> Artificial Sequence
<220>
   <223> Description of Artificial Sequence: Primer
<400> 10
   catcgctata gctgcgccaa aacagtttgg 30
<210> 11
   <211> 30
   <212> DNA
   <213> Artificial Sequence
<220>
   <223> Description of Artificial Sequence: Primer
<400> 11
   aactgttttg gattcagtat agcgatggcg 30
<210> 12
   <211> 30
   <212> DNA
   <213> Artificial Sequence
<220>
   <223> Description of Artificial Sequence: Primer
<400> 12
   cgccatcgct atactgaatc caaaacagtt 30
<210> 13
   <211> 30
   <212> DNA
   <213> Artificial Sequence
<220>
   <223> Description of Artificial Sequence: Primer
<400> 13
   aacgggggct cctgcgagga ccagctgcag 30
<210> 14
   <211> 30
   <212> DNA
   <213> Artificial Sequence
<220>
   <223> Description of Artificial Sequence: Primer
<400> 14
   ctgcagctgg tcctcgcagg agcccccgtt 30
<210> 15
   <211> 30
   <212> DNA
   <213> Artificial Sequence
<220>
   <223> Description of Artificial Sequence: Primer
<400> 15
   gggggctcct gccgcgacca gctgcagagc 30
<210> 16
   <211> 30
   <212> DNA
   <213> Artificial Sequence
<220>
   <223> Description of Artificial Sequence: Primer
<400> 16
   gctctgcagc tggtcgcggc aggagccccc 30
<210> 17
   <211> 30
   <212> DNA
   <213> Artificial Sequence
<220>
   <223> Description of Artificial Sequence: Primer
<400> 17
   gagctatatc tgcgagtgcc tgcctgcctt 30
<210> 18
   <211> 30
   <212> DNA
   <213> Artificial Sequence
<220>
   <223> Description of Artificial Sequence: Primer
<400> 18
   aaggcaggca ggcactcgca gatatagctc 30
<210> 19
   <211> 30
   <212> DNA
   <213> Artificial Sequence
<220>
   <223> Description of Artificial Sequence: Primer
<400> 19
   ggggcgcaat tgccagaccc ataaggatga 30
<210> 20
   <211> 30
   <212> DNA
   <213> Artificial Sequence
<220>
   <223> Description of Artificial Sequence: Primer
<400> 20
   tcatccttat gggtctggcaa ttgcgcccc 30
<210> 21
   <211> 26
   <212> DNA
   <213> Artificial Sequence
<220>
   <223> Description of Artificial Sequence: Primer
<400> 21
   gagcggacca aagagttttgg attagc 26
<210> 22
   <211> 26
   <212> DNA
   <213> Artificial Sequence
<220>
   <223> Description of Artificial Sequence: Primer
<400> 22
   gctaatccaa aactctttggt ccgctg 26
<210> 23
   <211> 26
   <212> DNA
   <213> Artificial Sequence
<220>
   <223> Description of Artificial Sequence: Primer
<400> 23
   gagcggacca aacagttttgg attagc 26
<210> 24
   <211> 26
   <212> DNA
   <213> Artificial Sequence
<220>
   <223> Description of Artificial Sequence: Primer
<400> 24
   gctaatccaa aactgtttggt ccgctc 26
<210> 25
   <211> 30
   <212> DNA
   <213> Artificial Sequence
<220>
   <223> Description of Artificial Sequence: Primer
<400> 25
   ctgcaaagac cagcagcagag ctatatctgc 30
<210> 26
   <211> 30
   <212> DNA
   <213> Artificial Sequence
<220>
   <223> Description of Artificial Sequence: Primer
<400> 26
   gcagatatag ctctgctgctg gtctttgcag 30
<210> 27
   <211> 30
   <212> DNA
   <213> Artificial Sequence
<220>
   <223> Description of Artificial Sequence: Primer
<400> 27
   ctgcaaagac cagtcccagag ctatatctgc 30
<210> 28
   <211> 30
   <212> DNA
   <213> Artificial Sequence
<220>
   <223> Description of Artificial Sequence: Primer
<400> 28
   gcagatatag ctctgggactg gtctttgcag 30
<210> 29
   <211> 29
   <212> DNA
   <213> Artificial Sequence
<220>
   <223> Description of Artificial Sequence: Primer
<400> 29
   cagagctata tctgcgactgc ctgcctgcc 29
<210> 30
   <211> 29
   <212> DNA
   <213> Artificial Sequence
<220>
   <223> Description of Artificial Sequence: Primer
<400> 30
   ggcaggcagg cagtcgcagat atagctctg 29
<210> 31
   <211> 28
   <212> DNA
   <213> Artificial Sequence
<220>
   <223> Description of Artificial Sequence: Primer
<400> 31
   cagagctata tctgctactgc ctgcctgc 28
<210> 32
   <211> 28
   <212> DNA
   <213> Artificial Sequence
<220>
   <223> Description of Artificial Sequence: Primer
<400> 32
   gcaggcaggc agtagcagata tagctctg 28
<210> 33
   <211> 42
   <212> DNA
   <213> Artificial Sequence
<220>
   <223> Description of Artificial Sequence: Primer
<400> 33
   gaacgggggc tcctgcgagga ccagcagcag agctatatct gc 42
<210> 34
   <211> 42
   <212> DNA
   <213> Artificial Sequence
<220>
   <223> Description of Artificial Sequence: Primer
<400> 34
   gcagatatag ctctgctgctg gtcctcgcag gagcccccgt tc 42